# EUROPEAN PATENT SPECIFICATION

(11) **EP 1 749 870 B1**
(45) Date of publication and mention of the grant of the patent: **14.05.2008**
(21) Application number: 05291676.4
(22) Date of filing: 04.08.2005
(51) Int. Cl.: C09K 11/06, C07C 229/54, C07C 235/16, C07C 237/04

(54) **Fluorescein-based compounds and their use for peptide synthesis**
Fluorescein-Derivate und ihre Verwendung für Peptidsynthese.
Dérivés de fluorescéine et leur utilisation pour la synthèse de peptide.

(43) Date of publication of application: 07.02.2007
(73) Proprietor: COMMISSARIAT A L'ENERGIE ATOMIQUE, 75015 Paris (FR)
(72) Inventor: Balakirev Maxim, 38000 Grenoble (FR); Burchak Olga, 38000 Grenoble (FR)
(74) Representative: Bolinches, Michel Jean-Marie

(56) References cited:
- EP-A- 0 567 841
- DATABASE CA [Online] CHEMICAL ABSTRACTS SERVICE, COLUMBUS, OHIO, US; ZHU, SHIXIONG ET AL: "Study of bulk morphology of polymer latex films using laser confocal fluorescence microscopy" XP002353538 retrieved from STN Database accession no. 1998:713988 & SCIENCE IN CHINA, SERIES B: CHEMISTRY , 41(5), 549-555 CODEN: SCBCFQ; ISSN: 1006-9291, 1998,
- DATABASE CHEMCATS [ONLINE] Chemical Abstracts Service, Columbus, Ohio, US; retrieved from STN XP002353539 & "ChemBridge Screening Library" 12 January 2005 (2005-01-12), CHEMBRIDGE CORPORATION , SAN DIEGO, CA, 92127 USA
- JISKOOT, WIM ET AL: "Preparation and application of a fluorescein-labeled peptide for determining the affinity constant of a monoclonal antibody-hapten complex by fluorescence polarization" ANALYTICAL BIOCHEMISTRY , 196(2), 421-6 CODEN: ANBCA2; ISSN: 0003-2697, 1991, XP002946153
- WAGNER, JUERGEN ET AL: "Synthesis of five enantiomerically pure haptens designed for in vitro evolution of antibodies with peptidase activity" BIOORGANIC & MEDICINAL CHEMISTRY , 4(6), 901-916 CODEN: BMECEP; ISSN: 0968-0896, 1996, XP002942286

## Description

The subject-matter of the present invention is related to new fluorescein derivatives, the method for producing such derivatives and their use for the synthesis of fluorogenic peptides and in particular protease substrates and peptide ligands.

Fluorescent dyes are widely employed in both qualitative and quantitative biological and chemical analysis as labels and staining reagents. Many xanthene derivatives are employed as fluorescent dye among which fluorescein and derivatives thereof including carboxyfluoresceins, aminofluoresceins, fluorescein isothiocyanates and the aminomethylfluoresceins can be cited. However, the low photostability and dependence of the fluorescence on pH are two most important drawbacks limiting biological applications of fluorescein analogues. Indeed, in fluorescence measurements such as immunofluorescence, dealing with small amount of fluorescent molecules and/or small sample volume, the irreversible destruction or photobleaching of the excited fluorophore becomes the factor limiting fluorescence detectability.

One obvious way to the modification of fluorescein derivatives is a conjugation of the carboxylic group on the phenyl ring with a nucleophile. However, such fonctionalization chemistry is complicated by tautomeric equilibrium between a locked non-fluorescent *spiro*-lacton form and an open fluorescent quinoid form as well as by ionic equilibrium of multiple (de)protonated forms of fluorescein (Klonis et al., J. Fluoresc., 1996, 6, 147-159 ; Martin et al., J. Luminesc., 1975, 10, 381-390 ; Orndorff et al., J. Am. Chem. Soc., 1927, 49, 1272-1280 ; Fompeydie et al., Bull. Soc. Chim. France, 1980, 459-465). This results in highly heterogeneous chemical composition, which depends on pH and solvent. Of notes, the multiplicity of fluorescein forms having different spectral properties also limits its application as a fluorescent probe (Tsien et al., Methods Cell. Biol., 1989, 30, 127-156). Two methods to avoid the tautomerization problem described in the literature include a coupling of the carboxylic group on the phenyl ring to secondary amines (Gao et al.; Anal. Chem., 2002, 74, 6397-6401 ; Nguyen et al., Org. Lett. 2003, 5, 3245-3248) and its esterification (Lohse et al., Bioconjugate Chem., 1997, 8 (4), 503-509 ; Adamczyk et al., Bioconjugate Chem., 1999, 10 (3), 544-547). While the formation of secondary amide of NNN'N'-tetrasubstituted rhodamines proceeds with a relatively high yield (Nguyen *et al*., previously cited), the reaction is less efficient with fluorescein and required additional activation step (Gao *et al*., previously cited). On the other hand, the esterification of the carboxylic group on the phenyl ring of fluorescein have been reported to give an excellent yield (Adamczyk *et al*., previously cited).

Adamczyk *et al*. (previously cited) discloses the synthesis of 3'-O-(carboxyalkyl)fluorescein methyl ester labels by esterification of fluorescein in methanol to provide with fluorescein methyl ester and coupling the hydroxyl function of the xanthinyl ring of the fluorescein methyl ester thus obtained with hydroxyalkyl benzyl esters. Then, after selective hydrolysis of the benzyl esters (without methyl ester cleavage), the desired 3'-O-(carboxyalkyl)fluorescein methyl ester labels are obtained. The fluorescein labels thus provided with are pH-independent from pH 4,8 to 9,4.

Gao *et al*. (previously cited) and US patent application 10/250,975 published as US 2004/0054195 disclose fluorescein derivatives termed "xanthamides". In particular, two derivatives are described : the first one containing a carboxymethyl ether at the 6-position on the xanthen ring and a secondary amide of dimethylamine on the carboxylic acid function of the phenyl ring and the second one containing a corresponding 6-methyl ether and a secondary amide of isonipecotic acid on the carboxylic acid function of the phenyl ring. These derivatives thus contain a single carboxyl group. By virtue of 6-O-alkylation with conversion of the carboxyl group to a secondary amide providing steric protection, such derivatives are photostable relatively to fluorescein and exhibit a pH-independent fluorescence.

However, the fluorescein derivatives disclosed in the prior art mentioned above are not appropriate for direct use in the synthesis of fluorescent peptides.

In this application field, Lohse *et al*. (previously cited) disclosed the synthesis of fluorescein-conjugated lysine monomers for solid phase synthesis of fluorescent peptides and PNA oligomers. Fluorescein ethyl ester was used to prepare a fluorescent mixed ester/ether 6-O-(carboxymethyl)-fluorescein ethyl ester thus conjugated to the amino group of lysine to give a Boc-protected fluorescein-conjugated lysine monomer. Removal of the Boc group, followed by reaction with Fmoc chloride, gave a Fmoc-protected monomer. These Boc- and Fmoc-protected fluorescein-conjugated lysines were readily incorporated into peptides and PNA oligomers during solid phase synthesis to give fluorescent products.

However, the compounds disclosed in this document are all lysine derivatives and this article does not shown any data related to the photostability of such derivatives.

Therefore, there is a need for the development of alternative fluorogenic labels, appropriate for the synthesis of fluorogenic peptides while not having the drawback of some derivatives disclosed in the prior art, and in particular being more photostable than fluorescein, having constant pH-independent fluorescence and not being subject to tautomerization. It is particularly desired to obtain labels which can be used in peptide synthesis, and notably in solid phase peptide synthesis in association with any amino-acid.

In a first aspect, the present invention relates to fluorescent compounds of formula (I): 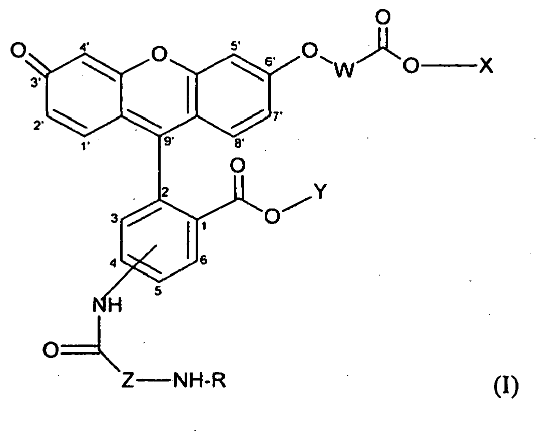 wherein :
W represents a di-radical selected from the group consisting of a linear, branched or cyclic C₁-C₁₂ alkyl, C₂-C₁₂ alkenyl, C₆-C₁₀ aryl, and C₆-C₁₂ alkylaryl or aralkyl,
X is chosen among the group consisting of H, a linear, branched or cyclic C₁-C₁₂ alkyl or C₂-C₁₂ alkenyl, a phenyl, a para-nitro phenyl and a benzyl,
Y represents a radical selected from the group consisting of a linear, branched or cyclic C₁-C₆ alkyl, C₂-C₆ alkenyl, a phenyl, a para-nitro phenyl, a benzyl, C₆-C₁₀ aryl and C₆-C₁₂ alkylaryl or aralkyl optionally containing one or several heteroatoms selected from the group consisting of O, N and S,
Z represents a di-radical selected from the group consisting of C₁-C₁₂ alkyl chains, wherein said chain may be interrupted by one or more of the following functionalities: an ether bridge -O- , an amino bridge -NH-, an ester function -O-CO-, an amide function -NH-CO-, a sulfur bridge -S-, a urea function -NH-CO-NH-, an oxycarbonyl function -O-CO-O-, C₆-C₁₀ aryl, C₆-C₁₀ heteroaryls optionally containing one or several heteroatoms selected from the group consisting of O, N and S and C₆-C₁₂ alkylaryl or aralkyl,
NH of the phenyl ring is at position 3 to 6 of the ring,
R is an amino protecting group, selected from the group consisting of : Fmoc, t-BuO-CO- (Boc), phenyl, benzyl, acetyl, benzoyl, 4-toluenesulfonyl, 4-methoxy-2,3,6-trimethylbenzenesulfonyl, trityl, 4-methoxytrityl, trifluoroacetyl, benzyloxycarbonyl, allyloxycarbonyl, 2-chlorobenzyloxycarbonyl,
and R, X and Y groups are selected so that R and X can be deprotected independently from each other and without deprotecting Y.

The compounds of the invention have the advantage of having on the phenyl ring a carboxylic acid group blocked by an appropriate protecting group Y which will not be removed when X and/or R are removed. So that the compounds of the invention are no subject to tautomerization.

On the other hand, and contrarily to the molecules disclosed in the prior art, the compounds of the invention have a carboxylic acid function, possibly protected by the X group, and an amino function, protected by the R group, said groups offering a protection which can be removed independently. This amino-acid functionalization of the fluorescein molecule permits the use of the compounds of formula (I) in peptide synthesis using usual methods of peptide synthesis, and especially these compounds can be used in solid phase automated peptide synthesis.

W is preferably a C₁-C₄ alkyl chain di-radical, even more preferably W is -CH₂-.

X can be preferably selected from H, C₁-C₆ alkyl, even more preferably from H and C₁-C₄ alkyl, like methyl, ethyl, n-propyl, isopropyl, n-butyl, isobutyl, ter-butyl. Preferably, X is ter-butyl.

Y is preferably C₁-C₆ alkyl, and even more preferably methyl or ethyl.

Z is preferably a chain comprising at least two carbon atoms. Z may be a C₂-C₈ alkyl chain, optionally comprising one or two functions selected from the group consisting of: an ether bridge, an amide function and an ester function. For example, Z can be selected from the group consisting of -CH₂-CH₂-, -CH₂-CH₂-CH₂-, -CH₂-(CH₂)₂-CH₂-, -CH₂-(CH₂)₃-CH₂-, -CH₂-(CH₂)₄-CH₂-, -CH₂-O-CH₂-CO-NH-CH₂-CH₂-, -CH₂-O-CH₂-CO-NH-(CH₂)₂-CH₂- and -CH₂-O-CH₂-CO-NH-(CH₂)₃₋CH₂-.

R is preferably a Fmoc group.

Preferred compounds of formula (I) are the following:

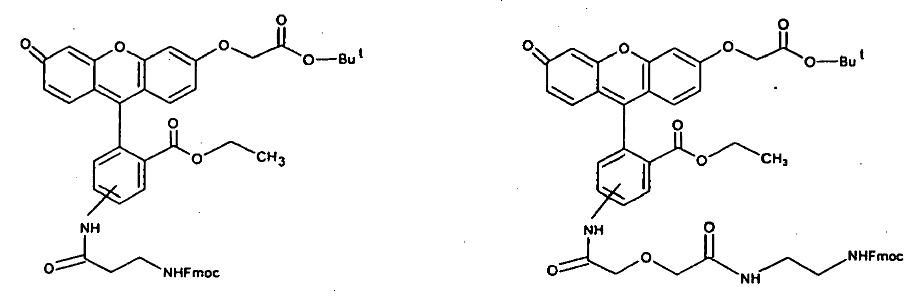

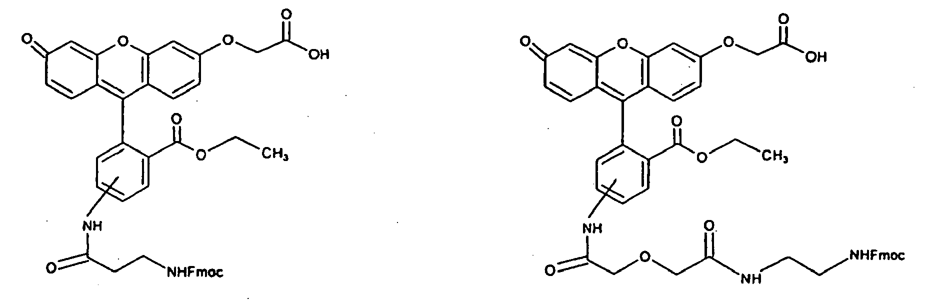

In a second aspect, the present invention relates to a method for producing the fluorescent compounds according to the invention. This method is carried out according to the following reaction scheme 1, wherein W, X, Y and Z have the same definition as in formula (I) above unless otherwise indicated. 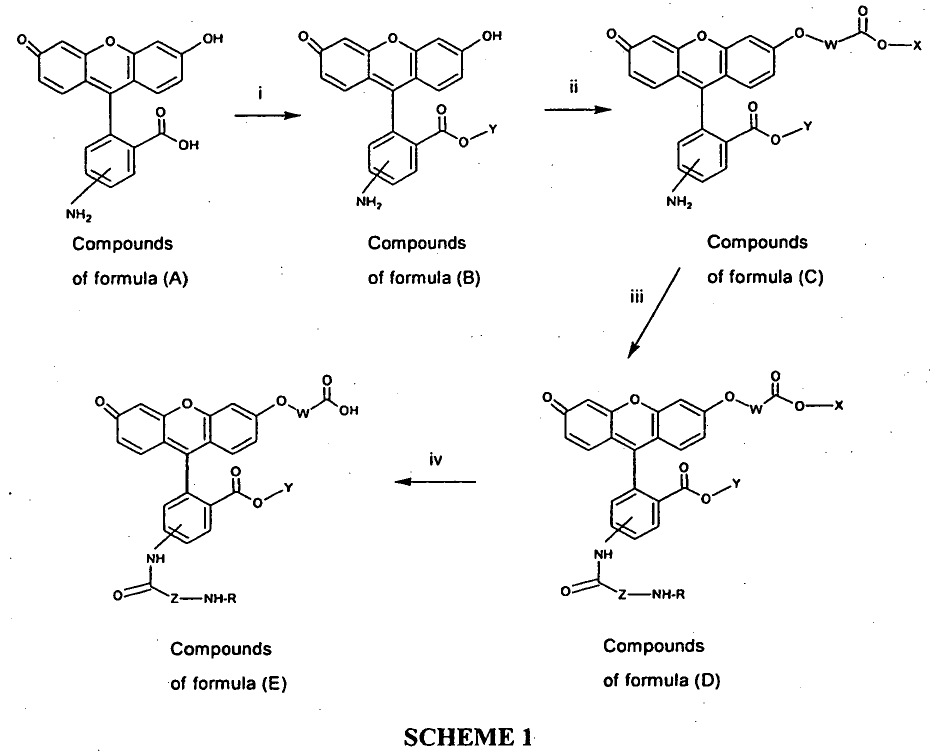
- Step i : the amino-fluoresceins (compounds of formula (A)) are commercially available molecules sold by Fluka, for example under the references 07980 and 07985. It should be noted, however, that there is a known cost-effective route for aminofluoresceins synthesis that involves condensation of 1,3-dihydroxybenzene with 3-nitrophthalic acid with subsequent reduction of the nitro group (Coons et al., J. Exp. Med., 1950, 91(1), 1-13 or 31-38; Boreskov et al., Bioorg. Khim., 1988, 14(3), 333-339; Patent Application GB 846674).

Compounds of formula (A) can be represented by the two following formulae:

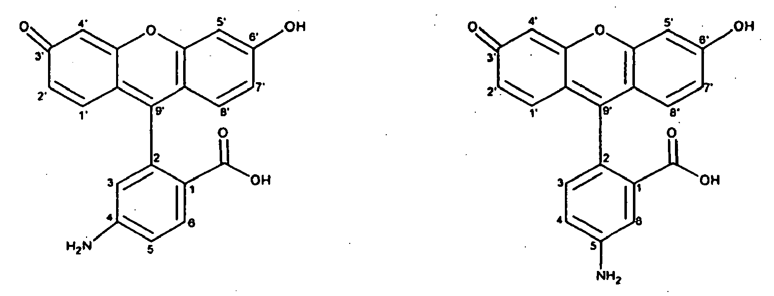

In solution, 4-amino-2-(6'-hydroxy-3'-oxo-3*H*-xanthen-9'-yl)-benzoic acid and 5-amino-2-(6'-hydroxy-3'-oxo-3*H*-xanthen-9'-yl)-benzoic acid are in equilibrium with their *spiro*-lacton tautomer, 6-amino-fluorescein and 5-amino-fluorescein respectively, responding to the following formulae:

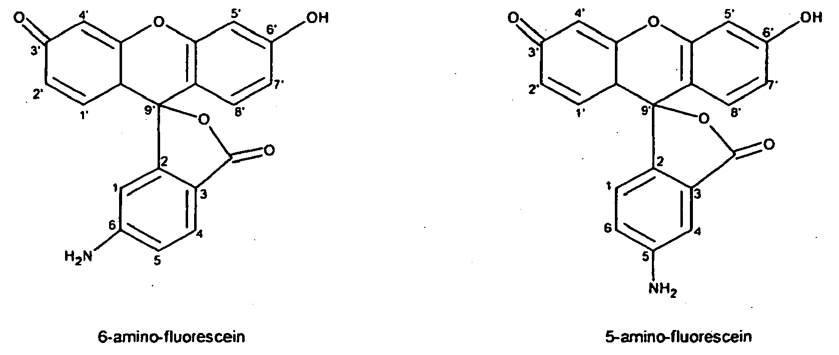

The amino-fluoresceins (compounds of formula (A)) are firstly esterified in refluxing alcohol Y-OH such as methanol or ethanol in acidic conditions, providing with the corresponding fluorescein ester (compounds of formula (B)).

According to a preferred feature of the invention, the esterification is carried out in ethanol, in presence of H₂SO₄ (for example using 5 mmol of H₂SO₄ for 1 mmol of amino-fluorescein).

The compounds thus obtained are optionally purified using any conventional method well known to the man skilled in the art and for example using chromatography on silica gel.

The compounds of formula (B) as depicted in scheme 1 are important intermediates in the synthesis of compounds of formula (I). They are new and constitute an other object of the instant invention.
- Step ii : a carboxylic acid moiety is introduced by alkylation of compounds of formula (B) with an alkyl halogeno-alkylcarboxylate group Hal-W-CO-OX, wherein W and X have the same definition as above for formula (I), except that X is not H, and Hal represents a halogen atom such as Cl, Br, I. Preferably, Hal is Br.

The reaction is conducted under basic conditions to provide with compounds of formula (C). The alkylation proceeds selectively on the 6'-OH function on the xanthen cycle and does not involve the amino group.
Step ii is preferably carried out in a polar aprotic solvent under basic conditions. This condition is the best method for alkylation of phenols in general and fluorescein particularly, for example as described by Crisp et al. in Tetrahedron Letters, 1997, 53, 1505-1522; Zaikova et al., Bioconjugate Chemistry, 2001, 12(2), 307-313). For example, preferred solvents to be used is dimethylformamide (DMF).

The compounds thus obtained are optionally purified using any conventional method well known to the man skilled in the art and for example using chromatography on silica gel.

Preferably, an alkylbromoacetate, and even more preferably t-butyl bromoacetate is used to carried out the alkylation of step ii.

The compounds of formula (C) as depicted in scheme 1 are important intermediates in the synthesis of compounds of formula (I). They are new and constitute an other object of the instant invention.

In addition, compounds of formula (C) could be used in peptide synthesis, according to a variant of the invention: a NH₂- protected amino acid can be reacted via its acid function with the NH₂ function of a compound of formula (C). The amino acid chain thus obtained can be further used in peptide synthesis.
- Step iii : compounds of formula (C) obtained from step ii are protected on their NH₂ function in a one step or a two steps reaction to provide with compounds of formula (D) corresponding to the particular compounds of formula (I) wherein X is not H.

When Z is an alkyl di-radical, R-NH-Z-COOH is reacted on compound of formula (C) in conditions such that a peptide link is created. Such conditions are well known to the man skilled in the art : the coupling may be made with the help of dicyclohexylcarbodiimide (DCC) or with the acid chloride R-NH-Z-COCl, or with the succinimide ester of R-NH-Z-COOH or any appropriate coupling method.

When Z is an alkyl chain interrupted by one of more function(s), the above disclosed method is adapted by the man skilled in the art. For example, when Z represents -CH₂-O-CH₂-CO-NH-(CH₂)ₓ-, x being an integer varying from 1 to 6, a suitable method consists in reacting the compound of formula C with diglycolic anhydride to obtain a derivative of formula (C) grafted with a -CO-CH₂-O-CH₂-CO-OH group on its amino function. Then, reacting this compound with a R-NH-(CH₂)ₓ₋NH₂ molecule in conditions to obtain a coupling between the amino and the carboxylic acid functions.

The compounds thus obtained are optionally purified using any conventional method well known to the man skilled in the art and for example using chromatography on silica gel.

According to other embodiments of the invention, step iii of the method for producing the fluorescent compounds can be carried out in a several steps procedure, said steps being adapted to the functions in the Z chain, according to methods known to the man skilled in the art.

The compounds obtained from the different stages are optionally purified using any conventional method well known to the man skilled in the art and for example using chromatography on silica gel.
- Step iv : the acidic function grafted on the 6'-hydroxy function of the xanthen ring of compounds of formula (D) is deprotected in order to obtain compounds of formula (E) corresponding to the particular compounds of formula (I) wherein X is H.

Any conventional deprotecting method can be used to carried out step iv, with the provision that it selectively deprotect the protective group X of compounds of formula (D). An hydrolysis under acidic conditions can be cited as such a deprotecting method, for example using trifluoroacetic acid (TFA) in dichloromethane (DCM) when X is a ter-butyl group.

When X has another functionality, the deprotection method of the carboxylic function on the 6'-OH position of the xanthen ring is adapted by the man skilled in the art.

In another aspect, the present invention relates to the use of the fluorescent compounds according to the present invention for the synthesis of fluorescent peptides.

In particular, the fluorescent compounds according to the present invention can be used for the synthesis of fluorogenic protease substrates or peptide ligands. The fluorescein compounds can also be incorporated into PNA oligomers or other compounds during solid phase synthesis.

The fluorescent compounds of formula (I) can be used directly in solid phase peptide synthesis.

When a compound of formula (I) is used for the preparation of a fluorogenic peptide, the steps illustrated on Scheme 2 may be followed.

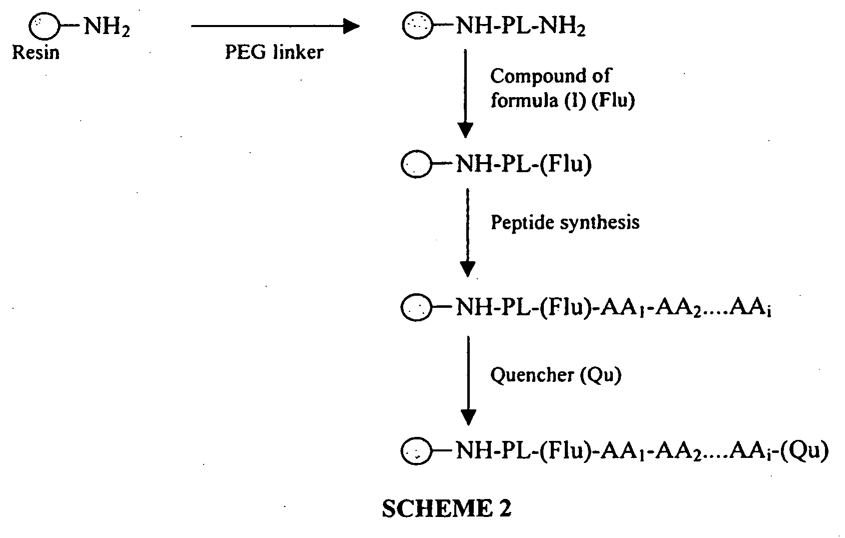

A resin with a NH₂ functionality is provided, like for example commercially available NH₂-resins based on Wang resin matrix, Rink resin matrix TentaGel matrix, PEGA matrix, Sasrin (Bachem) matrix etc. Otherwise, a linker containing Fmoc-protected NH₂ group can be introduced in the resin of choice. Then, the compound of formula (I) is grafted onto the resin using normal coupling method of peptide synthesis. Said compound is designated as (Flu) on Scheme 2.

Preferably, a NH₂-functionalised polar linker (designated as PL on Scheme 2) is coupled to the resin and the compound of formula (I) is grafted onto the NH₂-functionalised polar linker.

Indeed, preferred fluorescent compounds of formula (I) to be used for the peptide synthesis are those wherein there is a polar linker between the protected amino group and the fluorophore. In particular, the polar linker is preferably chosen among the group consisting of PEG linkers comprising from 2 to 300 PEG units. For example, PEG(7) represents a particular preferred polar linker to be used.

A compound responding to formula (II): 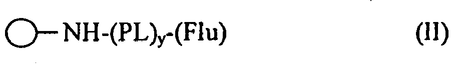 wherein 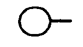
represents a resin,
PL represents a polar linker, like for example a PEG chain,
y is an integer selected from 0 and 1,
Flu is a compound of formula (I) grafted by its carboxylic acid function (case when X is a covalent link),
is new and is another object of the present invention.

Then, the peptide synthesis is done on the NH₂ group of the compound of formula (I) according to methods known to the man skilled in the art. Amino acids are named AA₁, AA₂, ....AAᵢ (with i = integer) on Scheme 2. After the peptide synthesis is finished, a quencher (marked (Qu) on Scheme 2) is grafted at the NH₂-terminal function of the peptide. The quencher may be any molecule whose absorbance spectrum overlaps with excitation/emission spectra of the Flu, or any paramagnetic molecule. Useful quenchers are known in the art; they are chosen, for instance, among the followings: Methyl Red, TAMRA (6-carboxytetramethylrhodamine) or dark quenchers; more precisely the following quenchers may be used Black Hole Quencher^{®} 1, 2 and 3 (Biosearch Technologies), Nanogold Particules^{®} (Nanoprobes), Eclipse Dark Quencher^{®} (Epoch Bioscience), Elle Quencher^{®} (Oswell), malachite green and QSY^{®} 7, QSY^{®} 9 and QSY^{®} 21 dyes (Molecular Probes). Preferably, the quencher is a Methyl Red chromophore.

The fluorogenic peptide grafted on the resin can be used directly in an enzymatic assay with a protease as illustrated on Scheme 3 representing the case of a hexapeptide with the use of a polar linker in the fluorogenic peptide.

Otherwise, the fluorogenic peptide can be cleaved from the resin, purified by chromatography (HPLC) and used in solution in homogeneous proteolysis assay.

Cutting of the peptide in two parts induces the emission of a bright fluorescence (Flu)* from the peptide part which is linked to the compound of formula (I) or (Flu).

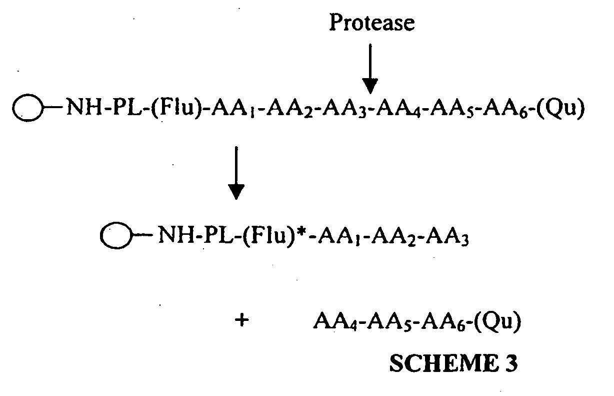

According to a variant, the peptide can be separated of the resin, by methods known to the man skilled in the art, before being used in enzymatic assays.

When the fluorogenic protease substrate is grafted onto resin beads, the measure of the distribution of bead fluorescence can be made by spotting the beads suspension on the surface of a microscope slide by using piezo-electric dispenser.

In addition, the beads can be pre-incubated with protease prior to spotting, or protease can be added directly to the arrayed beads.

Finally, the proteolysis of the bead-bound substrate can be measured by using micro-array scanner or fluorescent microscope.

The present invention will be further described by the examples below related to the synthesis of several fluorescent compounds according to the invention and the synthesis of a fluorogenic papain substrate based on one of these fluorescent compounds and Methyl Red as a fluorescence quencher, and the demonstration of its application for direct measuring papain activity on TentaGel beads. It is noted that these examples are given only for illustration purposes and that they are not intended to limit the invention.

### EXAMPLES

**Materials.** All chemicals were from Fluka unless indicated. Column chromatography was carried out on Merck Silica Gel 60. Thin layer chromatography was performed with Silica Gel 60 F₂₅₄ plates. ¹H NMR spectra were recorded on a Bruker (300 MHz); UV-Visible spectra were acquired with a Varian Cary 300 Scan spectrophotometer and fluorescence emission spectra with a Perkin-Elmer LS-50B luminescence spectrometer. The sciFLEXARRAYER piezoelectric dispensing system (Scienion) was used for beads spotting. Olympus BX51 microscope equipped with a Hamamatsu ORCA R camera and an Olympus U-RFL-T lamp was used for photobleaching study and beads imaging. Beads scanner images were obtained with a GeneTAC LS IV scanner (Genomic Solutions). Microscope images were analysed quantitatively using IMSTAR software (Khomyakova et al., Cell. Mol. Biol., 2004, 50, 217-224), and scanner images were analysed with Genepix Pro 4.0 software.

### EXAMPLE 1 : SYNTHESIS OF THE FLUORESCEIN COMPOUNDS ACCORDING TO THE INVENTION

### a) 5-amino-2-(6-hydroxy-3-oxo-3H-xanthen-9-yl)-benzoic acid ethyl ester (compound of formula B).

H₂SO₄ (266 µl, 5.0 mmol) was added dropwise to a suspension of 5-aminofluorescein (which is the *spiro*-lacton form of 5-amino-2-(6'-hydroxy-3'-oxo-3*H*-xanthen-9'-yl)-benzoic acid)(347 mg, 1.0 mmol) in 10 ml of EtOH at room temperature. After stirring at reflux for 24 h, EtOH was evaporated and the resulting mixture was diluted with CHCl₃. Solid NaHCO₃ was added to the solution until gas evolution ceased. The heterogeneous mixture was filtered, the organic layer was dried over Na₂SO₄, filtered, and concentrated under reduced pressure. The residue was submitted to chromatography on silica gel using EtOAc/hexane (1:1), MeOH/DCM (1:9), giving 326 mg (87%). ¹H NMR ((CD₃)₂SO, ppm): 0.80 (t, 3H, *J*=7.5 Hz), 3.87 (q, 2H, *J*=7.5 Hz), 5.86 (br.s, 2H), 6.55 (m, 4H), 6.90 (m, 3H), 7.04 (m, 1H), 7.33 (m, 1H).

### b) 4-amino-2-(6-hydroxy-3-oxo-3H-xanthen-9-yl)-benzoic acid ethyl ester (compound of formula B)

Such a compound was obtained with the same method as used for the synthesis of a) but starting from 6-aminofluorescein (which is the *spiro*-lacton form of 4-amino-2-(6'-hydroxy-3'-oxo-3*H*-xanthen-9'-yl)-benzoic acid) with yield 319 mg (85%). ¹H NMR ((CD₃)₂SO, ppm): 0.83 (t, 3H, *J*=7.5 Hz), 3.83 (q, 2H, *J*=7.5 Hz), 5.85 (br.s, 2H), 6.47 (m, 1H), 6.78 (m, 1H), 7.02 (m, 2H), 7.07 (m, 2H), 7.34 (m, 2H), 7.92 (m, 1H).

### c) 5-amino-2-(6-tert-butoxycarbonylmethoxy-3-oxo-3H-xanthen-9-yl)-benzoic acid ethyl ester (compound of formula C).

BrCH₂COOBu^{t} (215 mg, 1.1 mmol) was added to a mixture of compound **a)** (375 mg, 1.0 mmol) and K₂CO₃ (150 mg, 1.1 mmol) in 5 ml of DMF at room temperature. After stirring for 3 hours, the reaction mixture was diluted with H₂O and EtOAc and shaken. The organic layer was separated and washed with 1M NaHCO₃, brine, dried over Na₂SO₄ and concentrated under reduced pressure. The residue was submitted to chromatography on silica gel using EtOAc/hexane (1:1), MeOH/DCM (1:9), giving 455 mg (93%). ¹H NMR ((CD₃)₂SO, ppm): 0.82 (t, 3H, *J*=7.5 Hz), 1.42 (s, 9H), 3.88 (m, 2H), 4.85 (s, 2H), 5.87 (br.s, 2H), 6.20 (m, 1H), 6.37 (m, 1H), 6.82-7.05 (m, 5H), 7.14 (m, I H), 7.35 (s, 1H).

### d) 4-amino-2-(6-tert-butoxycarbonylmethoxy-3-oxo-3H-xanthen-9-yl)-benzoic acid ethyl ester (compound of formula C)

Such a compound was obtained by the same method as used for the synthesis of **c)** but starting from compound **b)** with yield 445 mg (91%). ¹H NMR ((CD₃)₂SO, ppm): 0.85 (t, 3H, *J*=7.5 Hz), 1.42 (s, 9H), 3.84 (m, 2H), 4.85 (s, 2H), 6.22 (m, 1 H), 6.30 (br.s, 2H), 6.37 (m, 1H), 6.44 (m, 1H), 6.77 (m, 1H), 6.88-6.96 (m, 3H), 7.15 (m, 1H), 7.90 (m, 1H).

### e) 5-acetylamino-2-(6-tert-butoxycarbonylmethoxy-3-oxo-3H-xanthen-9-yl)-benzoic acid ethyl ester (Model for compounds of formula D).

Ac₂O (47 µl, 0.5 mmol) was added to a solution of amine c) (98 mg, 0.2 mmol) and DIEA (88 µl, 0.5 mmol) in 5 ml of DCM at room temperature. After stirring overnight DCM was evaporated, the residue was diluted with H₂O and EtOAc, the mixture was stirred. The organic layer was separated and washed with 1M HCl, 1M NaHCO₃, brine, dried over Na₂SO₄ and concentrated under reduced pressure. The residue was subject to silica by chromatography using EtOAc/hexane (1:1), MeOH/DCM (1:9), giving 71 mg (67%). ¹H NMR ((CD₃)₂SO, ppm): 0.85 (t, 3H, *J*=7.5 Hz), 1.42 (s, 9H), 2.12 (s, 3H), 3.94 (m, 2H), 4.86 (s, 2H), 6.22 (m, 1H), 6.38 (m, 1H), 6.85-6.95 (m, 3H), 7.13 (m, 1H), 7.46 (m, 1H), 8.08 (m, 1H), 8.41 (s, 1H), 10.46 (s, 1H).

### f) 4-acetylamino-2-(6-tert-butoxycarbonylmethoxy-3-oxo-3H-xanthen-9-yl)-benzoic acid ethyl ester (Model for compounds of formula D)

Such a compound was obtained by the same method as used for the synthesis of **e)** but starting from amine **d)** with yield 74 mg (70%). ¹H NMR ((CD₃)₂SO, ppm): 0.85 (t, 3H, *J*=7.5 Hz), 1.41 (s, 9H), 2.08 (s, 3H), 3.85 (m, 2H), 4.93 (s, 2H), 6.41 (m, 1H), 6.54 (m, 1H), 7.95-7.02 (m, 3H), 7.29 (m, 1H), 7.72 (m, 1H), 7.88 (m, 1H), 8.19 (m, 1 H), 10.52 (s, 1H).

### g) 2-(6-tert-butoxycarbonylmethoxy-3-oxo-3H-xanthen-9-yl)-5-[3-(9H-fluoren-9-ylmethoxy-carbonylamino)-propionylamino]-benzoic acid ethyl ester (compound of formula D).

DCC (412 mg, 2.0 mmol) was added to a solution of fmoc-β-alanine (622 mg, 2.0 mmol) in 10 ml of DCM at room temperature, after stirring for 30 min, a solution of amine c) (489 mg, 1.0 mmol) in 5 ml of DCM was added to the mixture. After stirring during 2 days, DCM was evaporated, the residue was diluted with H₂O and EtOAc, the mixture was shaken. The organic layer was washed with 1M HCl, 1M NaHCO₃, brine, dried over Na₂SO₄ and concentrated under reduced pressure. The residue was submitted to chromatography on silica gel by using EtOAc/hexane (1:1), EtOAc, giving 352 mg (45%). ¹H NMR ((CD₃)₂SO, ppm): 0.85 (t, 3H, *J*=7.5 Hz), 1.41 (s, 9H), 2.55 (m, 2H), 3.35 (m, 2H), 3.96 (m, 2H), 4.21 (m, 1H), 4.60 (m, 2H), 4.92 (s, 2H), 6.18 (m, 1H), 6.22 (s, 1H), 6.38 (m, 1H), 6.49-6.61 (m, 3H), 6.69 (m, 1H), 6.96 (m, 1H), 7.15 (m, 1H), 7.26-7.39 (m, 4H), 7.64 (m, 2H), 7.86 (m, 1H), 7.88 (m, 2H), 10.48 (s, 1H).

### h) 2-(6-tert-butoxycarbonylmethoxy-3-oxo-3H-xanthen-9-yl)-4-[3-(9H-fluoren-9-ylmethoxycarbo-nylamino)-propionylaminol-benzoic acid ethyl ester (compound of formula D)

Such a compound was obtained by the same method as used for the synthesis of **g)** but starting from amine **d**) with yield 375 mg (48%). ¹H NMR ((CD₃)₂SO, ppm): 0.86 (t, 3H, *J*=7.5 Hz), 1.42 (s, 9H), 2.38 (m, 2H), 3.39 (m, 2H), 3.92 (m, 2H), 4.19 (m, 1H), 4.58 (m, 2H), 4.85 (s, 2H), 6.21 (m, 1H), 6.30 (s, 1H), 6.42 (m, 1H), 6.78 (m, 1H), 6.84-6.97 (m, 3H), 7.17 (m, 1H), 7.23 (m, 2H), 7.25-7.42 (m, 4H), 7.64 (m, 2H), 7.87 (m, 2H), 10.53 (s,1H).

### i) 2-(6-tert-butoxycarbonylmethoxy-3-oxo-3H-xanthen-9-yl)-5-(2-{[2-(9H-fluoren-9-ylmethoxy-carbonylamino)-ethylcarbamoyl]-methoxy}-acethylamino]-benzoic acid ethyl ester (compound of formula D).

### Synthesis of the intermediate acid 1 : 2-(6-tert-butoxycarbonylmethoxy-3-oxo-3H-xanthen-9-yl)-5-(2-carboxymethoxy-acetyl-amino)-benzoic acid ethyl ester.

DIEA (35 µl, 0.2 mmol) was added to a solution of amine c) (98 mg, 0.2 mmol), diglycolic anhydride (23 mg, 0.2 mmol) and DMAP (24 mg, 0.2 mmol) in 5 ml of DCM at room temperature. After stirring overnight, DCM was evaporated, the residue was diluted with H₂O and EtOAc, the mixture was shaken. The organic layer was separated, washed with 1M HCl, brine, dried over Na₂SO₄ and concentrated under reduced pressure. The residue was purified by chromatography on silica gel by using DCM, AcOH/MeOH/DCM (1:5:100), giving 91 mg (75%). ¹H NMR ((CD₃)₂SO, ppm): 0.83 (t, 3H, *J*=7.5 Hz), 1.42 (s, 9H), 3.95 (m, 2H), 4.22 (s, 2H), 4.24 (s, 2H), 4.87 (s, 2H), 6.23 (m, 1H), 6.39 (m, 1H), 6.84-6.92 (m, 3H), 7.17 (m, 1H), 7.44 (m, 1 H), 8.12 (m, I H), 8.52 (m, 1 H), 10.42 (s, 1H).

### Synthesis of 2-(6-tert-butoxycarbonylmethoxy-3-oxo-3H-xanthen-9-yl)-5-(2-{[2-(9H-fluoren-9-ylmethoxy-carbonylamino)-ethylcarbamoyl]-methoxy}-acetylamino]-benzoic acid ethyl ester (compound of formula D).

DCC (41 mg, 0.2 mmol) was added to a solution of the **intermediate acid 1** (121 mg, 0.2 mmol) in 5 ml of DCM at room temperature, after stirring for 30 min, a solution of Fmoc-ethylendiamine (56 mg, 0.2 mmol) in 2 ml of DCM was added to the mixture. After stirring overnight, DCM was evaporated, the residue was diluted with H₂O and EtOAc, the mixture was shaken. The organic layer was separated, washed with 1M HCl, 1M NaHCO₃, brine, dried over Na₂SO₄ and concentrated under reduced pressure. The residue was purified by chromatography on silica gel using EtOAc/hexane (1:1), EtOAc, MeOH/DCM (1:20), giving 101 mg (58%). ¹H NMR ((CD₃)₂SO, ppm): 0.85 (t, 3H, *J*=7.5 Hz), 1.42 (s, 9H), 3.11 (m, 2H), 3.19 (m, 2H), 3.92 (m, 2H), 4.08 (s, 2H), 4.16 (m, 1 H), 4.21 (s, 2H), 4.28 (m, 2H), 4.86 (s, 2H), 6.24 (m, 1H), 6.37 (m, 1H), 6.80-6.97 (m, 3H), 7.19 (m, 1H), 7.27-7.42 (m, 4H), 7.65 (m, 2H), 7.64 (m, 1H), 7.88 (m, 2H), 7.82 (m, 1H), 8.05 (m, 1H), 10.49 (s, 1H).

### j) 2-(6-tert-butoxycarbonylmethoxy-3-oxo-3H-xanthen-9-yl)-4-(2-{[2-(9H-fluoren-9-ylmetho-xycarbonylamino)-ethylcarba-moyl]-methoxy}-acethylamino]-benzoic acid ethyl ester (compound of formula D)

An intermediate acid 2 (2-(6-tert-butoxycarbonylmethoxy-3-oxo-3H-xanthen-9-yl)-4-(2-carboxymethoxy-acetylamino)-benzoic acid ethyl ester) was obtained by the same method as used for the synthesis of the intermediate acid 1 mentioned above but starting from amine **d**) with yield 85 mg (70%). ¹H NMR ((CD₃)₂SO, ppm): 0.87 (t, 3H, *J*=7.5 Hz), 1.42 (s, 9H), 3.93 (m, 2H), 4.11 (s, 2H), 4.18 (s, 2H), 4.86 (s, 2H), 6.24 (m, 1H), 6.39 (m, 1H), 6.83-6.96 (m, 3H), 7.19 (m, 1H), 7.76 (m, 1H), 8.03 (m, 1H), 8.18 (m, 1H), 10.48 (s, 1H).
The synthesis of 2-(6-tert-butoxycarbonylmethoxy-3-oxo-3H-xanthen-9-yl)-5-(2-{[2-(9H-fluoren-9-ylmethoxy-carbonylamino)-ethylcarbamoyl]-methoxy}-acethylamino]-benzoic acid ethyl ester (compound of formula D) was obtained by the same method as used for the synthesis of i) but **using the intermediate acid 2** with yield 111 mg (64%). ¹H NMR ((CD₃)₂SO, ppm): 0.86 (t, 3H, *J*=7.5 Hz), 1.42 (s, 9H), 3.07 (m, 2H), 3.15 (m, 2H), 3.91 (m, 2H), 4.04 (s, 2H), 4.16 (m, 1H), 4.19 (s, 2H), 4.26 (m, 2H), 4.85 (s, 2H), 6.22 (m, 1H), 6.48 (m, 1H), 6.82-6.95 (m, 3H), 7.17 (m, 1H), 7.27-7.41 (m, 4H), 7.64 (m, 2H), 7.77 (m, 1H), 7.86 (m, 2H), 8.00 (m, 1H), 8.16 (m, 1H), 10.49 (s, 1H).

### k) 5-acetylamino-2-(6-carboxymethoxy-3-oxo-3H-xanthen-9-yl)-benzoic acid ethyl ester (Model for compounds of formula E : dye 15).

TFA (2.0 g, 1.3 ml) was added to a solution of ester e) (53 mg, 0.1 mmol) in DCM (2.0 g, 1.5 ml) at room temperature. After 3 hours DCM and TFA were evaporated, the residue was diluted with Et₂O, formed solid precipitate was filtered off, washed with Et₂O and dried to afford 45 mg of the desired acid (95%). NMR ((CD₃)₂SO, ppm): 0.85 (t, 3H, *J*=7.5 Hz), 2.12 (s, 3H), 3.96 (m, 2H), 4.87 (s, 2H), 6.22 (m, 1H), 6.39 (m, 1H), 6.84-6.92 (m, 3H), 7.17 (m, 1H), 7.40 (m, 1H), 8.04 (m, 1H), 8.40 (s, 1H), 10.45 (s, 1H).

### l) 4-acetylamino-2-(6-carboxymethoxy-3-oxo-3H-xanthen-9-yl)-benzoic acid ethyl ester (Model for compounds of formula E : dye 16)

Such a compound was obtained by the same method as used for the synthesis of **k)** but starting from ester **f)** with yield 44 mg (93%). ¹H NMR ((CD₃)₂SO, ppm): 0.83 (t, 3H, *J*=7.5 Hz), 2.10 (s, 3H), 3.83 (m, 2H), 4.93 (s, 2H), 6.41 (m, 1H), 6.55 (m, 1H), 6.95-7.02 (m, 3H), 7.29 (m, 1H), 7.72 (m, 1H), 7.89 (m, 1H), 8.19 (m, 1H), 10.52 (s, 1H).

### m) 2-(6-carbonylmethoxy-3-oxo-3H-xanthen-9-yl)-5-[3-(9H-fluoren-9-ylmethoxycarbonyl-amino)-propionylamino]-benzoic acid ethyl ester (compound of formula E).

TFA (2.0 g, 1.3 ml) was added to a solution of ester g) (782 mg, 1.0 mmol) in DCM (2.0 g, 1.5 ml) at room temperature. After 3 hours, DCM and TFA were evaporated, the residue was diluted with Et₂O, formed solid precipitate was filtered off, washed with Et₂O and dried to afford 704 mg of the desired acid (97%). ¹H NMR ((CD₃)₂SO, ppm): 0.85 (t, 3H, *J*=7.5 Hz), 2.58 (m, 2H), 3.39 (m, 2H), 3.93 (m, 2H), 4.32 (m, 1H), 4.66 (m, 2H), 4.87 (s, 2H), 6.21 (m, 1H), 6.24 (s, 1H), 6.40 (m, 1H), 6.50-6.59 (m, 3H), 6.69 (m, 1H), 6.70 (m, 1H), 7.15 (m, 1H), 7.26-7.39 (m, 4H), 7.64 (m, 2H), 7.88 (m, 1H), 7.89 (m, 2H), 10.49 (s, 1H).

### n) 2-(6-carbonylmethoxy-3-oxo-3H-xanthen-9-yl)-4-[3-(9H-fluoren-9-ylmethoxycarbonyl-amino)-propionylamino]-benzoic acid ethyl ester (compound of formula E)

Such a compound was obtained by the same method as used for the synthesis of **m)** but starting from ester **h**) with yield 690 mg (95%). ¹H NMR ((CD₃)₂SO, ppm): 0.85 (t, 3H, *J*=7.5 Hz), 2.36 (m, 2H), 3.23 (m, 2H), 3.92 (m, 2H), 4.25 (m, 1H), 4.51 (m, 2H), 4.85 (s, 2H), 6.21 (m, 1H), 6.33(s, 1H), 6.44 (m, 1H), 6.80 (m, 1H), 6.83-6.95 (m, 3H), 7.15 (m, 1H), 7.22 (m, 2H), 7.26-7.42 (m, 4H), 7.64 (m, 2H), 7.85 (m, 2H), 10.50 (s, 1H).

### o) 2-(6-carbonylmethoxy-3-oxo-3H-xanthen-9-yl)-5-(2-{[2-(9H-fluoren-9-ylmethoxycarbonyl-amino)-ethylcarbamoyl]-methoxy}-acethylamino]-benzoic acid ethyl ester (compound of formula E).

TFA (2.0 g, 1.3 ml) was added to a solution of ester i) (87 mg, 0.1 mmol) in DCM (2.0 g, 1.5 ml) at room temperature. After 3 hours, DCM and TFA were evaporated, the residue was diluted with Et₂O, formed solid precipitate was filtered off, washed with Et₂O and dried to afford 77 mg of the desired acid (95%). ¹H NMR ((CD₃)₂SO, ppm): 0.85 (t, 3H, *J*=7.5 Hz), 3.07 (m, 2H), 3.15 (m, 2H), 3.95 (m, 2H), 4.04 (s, 2H), 4.16 (m, 1H), 4.19 (s, 2H), 4.26 (m, 2H), 4.85 (s, 2H), 6.32 (m, 1H), 6.43 (m, 1H), 6.94-7.00 (m, 3H), 7.26 (m, 1H), 7.24-7.36 (m, 4H), 7.64 (m, 2H), 7.78 (m, 1H), 7.86 (m, 2H), 8.01 (m, 1H), 8.28 (m, 1H), 10.49 (s, 1H).

### p) 2-(6-carbonylmethoxy-3-oxo-3H-xanthen-9-yl)-4-(2-{[2-(9H-fluoren-9-ylmethoxycarbonyl-amino)-ethylcarbamoyl]-methoxy}-acethylamino]-benzoic acid ethyl ester (compound of formula E)

This compound was obtained by the same method as used for the synthesis of o) but starting from ester j) with yield 73 mg (90%). ¹H NMR ((CD₃)₂SO, ppm): 0.87 (t, 3H, *J*=7.5 Hz), 3.05 (m, 2H), 3.15 (m, 2H), 3.93 (m, 2H), 4.02 (s, 2H), 4.18 (m, 1H), 4.19 (s, 2H), 4.25 (m, 2H), 4.86 (s, 2H), 6.42 (m, 1H), 6.53 (m, 1H), 6.93-6.99 (m, 3H), 7.25 (m, 1H), 7.29-7.40 (m, 4H), 7.64 (m, 2H), 7.80 (m, 1H), 7.85 (m, 2H), 7.99 (m, 1H), 8.18 (m, 1H), 10.51 (s, 1H).

### EXAMPLE 2 : PHYSICO-CHEMICAL CHARACTERIZATION OF THE FLUORESCEIN COMPOUNDSS ACCORDING TO THE INVENTION.

The compounds **k**) (dye 15) and **l)** (dye 16) of formula E were synthetized as mentioned in Example 1 and used as a model of the compounds of formula (I) with regards to the photochemical properties of the synthesized fluorescein compounds. Indeed, the fluorescein compounds according to the invention **m)** to **p)** have the same organization of the fluorophore core as the model compounds **k)** and **l),** and are thus expected to have similar photochemical characteristics.

### a) UV absorbance spectrum

Standard UV-visible and fluorescence spectra were recorded in 50 mM sodium tetraborate buffer pH 9.8 at 0.5-10 µM fluorescent compounds concentration. The fluorescence quantum yield of the fluorescent compounds was determined as described by Adamczyk *et al*. (previously cited) using alkaline solution of fluorescein as a reference (φ=0.92) (Lohse *et al*. ; Adamczyk *et al*. ; Klonis *et al*. and Martin *et al*., all previously cited).

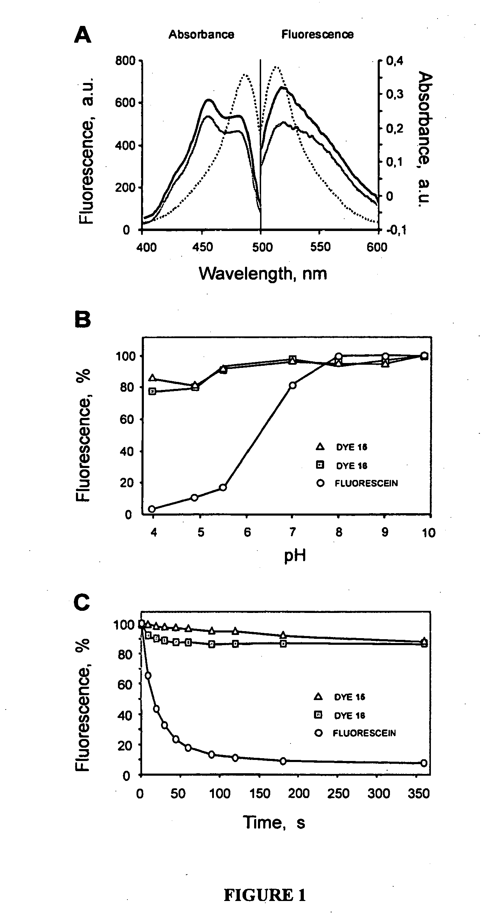

As shown in **Figure 1A** illustrating the absorbance and fluorescence spectra of compound **k)** (grey line), compound **l)** (black line) and fluorescein (dotted grey line), the UV-visible absorbance spectra of both compounds **k)** and **l)** exhibit two absorbance maxima at 456 and 481 nm. The maximum at 481 nm corresponds to the absorbance maximum of fluorescein at 490 nm suggesting that the same light sources can be used for excitation of these fluorophores. The shorter wavelength maximum at 456 nm is characteristic of 3-O-fluorescein ethers (He et al., Colloids and Surfaces A: Physicochemical and Engineering Aspects, 1998, 142, 49-57) and has been observed in absorbance spectra of various fluorescein ethers having 2'-ester (Lohse *et al*. ; Adamczyk *et al*., previously cited) or 2'-secondary amide residues (Goa *et al*., previously cited).

The molar extinction coefficients determined at 456 nm for **k)** (2.2 x 10⁴ L·mol⁻¹·cm⁻¹) and for **l)** (2.3 x 10⁴ L·mol⁻¹·cm⁻¹) are also close to reported values for 3-O-alkyl fluorescein 2'-esters (2.0-3.5 x 10⁴ L·mol⁻¹·cm⁻¹, Adamczyk *et al*. (previously cited)). This means that the presence of the 4- or 5-amido groups does not significantly affect the electronic properties of 3-O-alkyl fluoresceins.

The fluorescence emission spectra of compounds **k)** and **l)** are broadened and redshifted (λₘₐₓ=520 nm) relative to that of fluorescein (λₘₐₓ=512 nm). The fluorescence quantum yields determined for **k)** (φ=0.19) and for **l)** (φ=0.18) were notably lower than the quantum yield of fluorescein dianion (φ=0.92-0.93) (Klonis *et al*. ; Martin *et al*., previously cited ; Flemming et al., J. Am. Chem. Soc., 1977, 99, 4306-4311), and were consistent with the values reported for other 3-O-alkyl fluorescein ethers (φ=0.18-0.31) (Lohse *et al.* ; Adamczyk *et al*., previously cited).

### b) pH dependency

To measure the pH effect on fluorescence, 50 nM of fluorescent compounds **k)** (dye 15) or **l)** (dye 16) solution in 50 mM sodium tetraborate buffer (pH 9.8) was slowly titrated with HCl, aliquots were taken between pH 9.8 and 4, and fluorescence was measured at λ_{exc} 488 nm/ λₑₘ 515 nm.

As shown in **Figure 1B** illustrating pH-dependence of the fluorescence intensity of compound **k**) (dye 15) (Δ), compound **l)** (dye 16) (■) and fluorescein (○), the fluorescence and the quantum yields determined for compounds **k**) (dye 15) and **l)** (dye 16) are virtually pH-independent throughout the range of pH 4.0-10.0. This is explained by the absence of ionic equilibrium in 3-O-alkyl fluorescein derivatives trapped in a tautomeric quinoid form by 2'-esterification. The stability of 2'-ester toward an alkaline hydrolysis was also checked by following the absorbance and fluorescence spectra. No significant changes were observed after incubation of the fluorophores **k)** (dye 15) and **l)** (dye 16) for several hours in aqueous pH 9.8 buffer. The pH independence exhibited by these fluorophores is advantageous in applications requiring quantification and comparison of fluorescence intensity in different environments, the feasible examples being the fluorescence measurement in acidic intracellular compartments (Falck et al., J. Am. Chem. Soc., 1981, 103, 7396-7398 ; Galloway et al., Proc. Natl. Acad. Sci. U.S.A., 1983, 80, 3334-3338), and the studies of the pH-dependence of enzymatic activity with fluorogenic substrates (Andrews et al., Biochemistry, 1980, 19, 5494-5000 ; Fiorucci et al., FEBS Lett., 1997, 408, 85-88).

### c) Photostability

Super-Teflon glass slides (VWR International) carrying 40 circular wells of 2 mm diameter, separated by 30 µm-thick Teflon coating were used in dye photostability study. A well was filled with 1 µl of 5 µM fluorescein compounds **k**) (dye 15) or **l)** (dye 16) solution in 50 mM sodium tetraborate buffer pH 9.8 and was enclosed with a coverslip.

The entire well volume was irradiated under the microscope with standard "fluorescein" filters set. The intensity delivered by the source on the surface of the slide was measured with a COHERENT LaserCheck probe, and found to be ~6mW/cm² at 488 nm. Well images were recorded regularly with IMSTAR software and mean fluorescence intensity was calculated. The snapshots took 8-120 ms, that was considerably shorter than the characteristic time of photobleaching kinetics t_{1/2}> 14 s.

For comparison of the photostability of fluorophores under the condition of signal acquisition with fluorescent microscope, the sample was placed in a well on the surface of a glass slide that contains 40 circular wells of 2 mm diameter, separated by 30 µm-thick Teflon coating. The well was then enclosed with a coverslip producing a cylindrical sample chamber of ~380 nl volume. The slide was mounted on the microscope stage and the well was irradiated with standard "fluorescein" filters set. In order to prevent a heterogeneous photobleaching and associated macrodiffusion phenomena, the objective was adjusted to irradiate an entire sample chamber. The resulted light intensity at the chamber level was measured to be ~6 mW/mm². At different times the fluorescence images of the well were taken and mean fluorescence intensity was calculated using IMSTAR software (Khomyakova *et al*., previously cited).

As seen in **Figure 1C** illustrating the dye photobleaching study of compound **k**) (dye 15) (Δ), compound **l)** (dye 16) (■) and fluorescein (○), under these conditions fluorescein was rapidly bleached with characteristic time of 50% decay t_{1/2}~14s. Compounds **k)** (dye 15) and **l)** (dye 16) demonstrated remarkable photostability: only ~10% deacrease in fluorescence was observed for both fluorophores after 6 min of irradiation.

### EXAMPLE 3 : SOLID PHASE SYNTHESIS OF FLUOROGENIC SUBSTRATE USING A FLUORESCEIN COMPOUND ACCORDING TO THE INVENTION.

To demonstrate the utility of the synthesized fluorophores according to the invention for the solid phase synthesis of fluorogenic substrates, a fluorogenic substrate for cysteine protease papain has been designed.

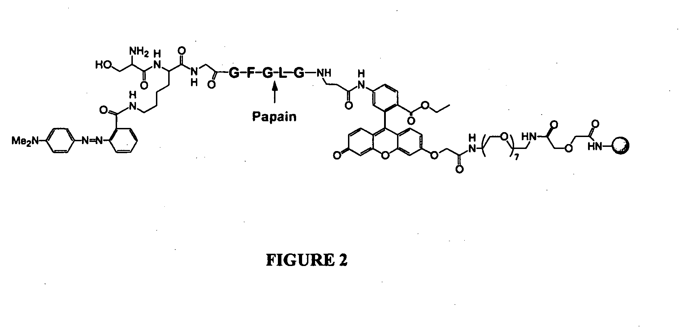

The substrate comprised of Fmoc-protected fluorescent compound **n)** and Methyl Red (MR) chromophore separated by the peptide sequence GGFGLG. that has been described as a good papain substrate with a cleavage being at the G-L bond (Leon et al., Bioorg. Med. Chem. Lett., 1998, 8, 2997-3002). The specificity for the papain is determined by the phenylalanine at the P2 position and the leucine at the position P1' (Schechter et al., Biochem. Biophys. Res. Commun., 1968, 32, 898-902). The MR dye has been shown to be well suited to quench the fluorescence of fluorescein derivatives (McKeen et al., Org. Biomol. Chem. 2003, 1, 2267-2275). The substrate was synthesized by Fmoc-chemistry on TentaGel resin (20 µm beads). For reasons of simplicity and potential high-throughput applications we have evaluated the validity of on-bead protease screening. The major concern of such heterophase screening assay is the permeability of resins to biomolecules, which determines the accessibility of the resin-bound substrate for the enzymes. Although TentaGel resin has been shown not to be optimal for on-bead enzymatic screening (Leon *et al*., previously cited), it has some important advantages such as excellent swelling properties in organic and aqueous media, mechanical stability and narrow size distribution (Olivos et al., Chembiochem 2003, 4, 1242-1245 ; Lam et al., Chem. Rev. 1997, 97, 411-448). Furthermore, a polyethylene glycol linker PEG(7) has been introduced between the peptide and TentaGel functional group rendered the fluorogenic substrate more accessible for protease cleavage. The fluorophore **n)** was coupled first after the linker followed by peptide sequence and the quencher MR, which was coupled to the side chain of the lysine at the end of the synthesis, as illustrated by **Figure 2**. In figure 2, the first G of the peptide sequence is represented by -NH-CH₂-CO.

### a) Synthesis of the substrate

The substrate was synthesized semi-manually on TentaGel S NH2 resin (20 µm beads, 0.25 mmol/g) using Bohdan MiniBlock synthesizer (Mettler Toledo). The resin (100 mg, 0.025 mmol) preswollen for 3 h in dry DCM was re-suspended in 2 ml of dry DCM, and the solution of Boc-PEG-acid (n=7) (44 mg, 0.075 mmol), diisopropylcarbodiimide (DIC) (16 mg, 0.125 mmol), HOBt (17 mg, 0.125 mmol) and DMAP (3 mg, 0.025 mmol) in 0.5 ml of dry DMF was added. The reaction was shaken overnight at room temperature. The resin was washed with dry DMF (3x 3 ml) and dry DCM (3x 3 ml) and treated with a mixture of acetic anhydride (18 µl, 0.2 mmol), pyridine (16 µl, 0.2 mmol) in 2 ml of dry DMF for 2 hours. As revealed by ninhidrin test, this procedure capped all non-reacted amino groups remained on the resin. After thorough washing with dry DMF and dry DCM, the resin was deprotected with 20% TFA in DCM.

The resulted TentaGel-PEG(7)-NH2 resin was used for substrate synthesis by standard Fmoc protocol (Chan *et al*., Oxford University Press: New York, 2002). Ten fold excess of Fmoc-protected compounds for 2 hours were used in each coupling cycle to ensure the completion of peptide coupling reaction (three fold excess overnight for Fmoc-protected fluorescent amino-acid **n)**. Each successive coupling was verified by ninhydrin test and by measuring resin weight. Prior to MR coupling, the synthesized sequence was confirmed by Edman sequencing. In order to attach MR, Nε-Boc lysine was introduced in the peptide chain. The Nε-Boc protecting group was removed with 20% TFA in DCM prior final Nα-Fmoc deprotection. The resin was resuspended in 2 ml of dry DCM, and was treated with a solution of MR (34 mg, 0.125 mmol), DIC (16 mg, 0.125 mmol), HOBt (17 mg, 0.125 mmol) and DMAP (3 mg, 0.025 mmol) in 0.5 ml of dry DMF. The reaction was shaken for 6 hours at room temperature. The resin was washed thoroughly with dry DMF and dry DCM and fully deprotected with 50% TFA in DCM.

### b) On-Bead Proteolysis Assay

Regardless of the biochemical model used, the on-bead screening assay requires methods for determination and analysis of the "positive" beads. In this example, the peptide sequence susceptible to proteolysis should be cleaved from the solid phase support thereby releasing the MR quenching moiety and resulting in an enhancement of the fluorescence of the resin-bound fluorophore. Therefore, to detect a proteolytic activity, the fluorescent proteolized beads should be distinguished from the control beads, which have not been exposed to a protease. functioning

The beads were swollen overnight in 0.4 M sodium phosphate buffer (pH 6.8), centrifuged and re-suspended in the same buffer containing 8 mM DTT and 4 mM EDTA (Szabelski et al., Acta Biochim. Pol. 2001, 48, 995-1002). The samples of beads suspension was incubated with 0.5 µM papain for 2 hours, then washed and examined for fluorescence. For inhibitor assays, 5 µM stock papain solution was pre-incubated with 1 mM N-ethylmaleimide (NEM) for 15 min at 37°C before adding to beads.

For beads imaging, the beads samples were spotted in the wells on Super-Teflon glass slides by using piezo-electric dispenser and were enclosed with coverslips. The 40-wells patterned glass slides with 30 µm-thick Teflon frame were used to delimit the bead solution, although higher density format (several thousands hydrophilic wells patterned on a perfluorated glass surface) can also be used. To avoid the clogging of the piezoelectric pipette, the beads were maintained in suspension by sonication pulses. Under these conditions rather homogeneous dispense of the beads 5-500 per droplet, depending on the bead concentration, can be achieved. The measure of the distribution of bead fluorescence has been performed in arrayed droplets without covering, but glycerol should be added to the beads suspension to prevent evaporation.

The proteolysis of the bead-bound substrate is measured by using microarray scanner or fluorescent microscope. The scanning were performed on GeneTAC LS IV scanner using a 488 nm argon ion laser and a 512 nm emission filter (512BP). The usual instrument settings were gain=26.0, black=1.5, and scanning resolution of 5 µm. The obtained images were analyzed with Genepix Pro 4.0 software. Microscope measurements were performed on a Olympus BX51 fluorescent microscope with standard "fluorescein" filters set. A random population of 200 beads was analyzed for each sample.

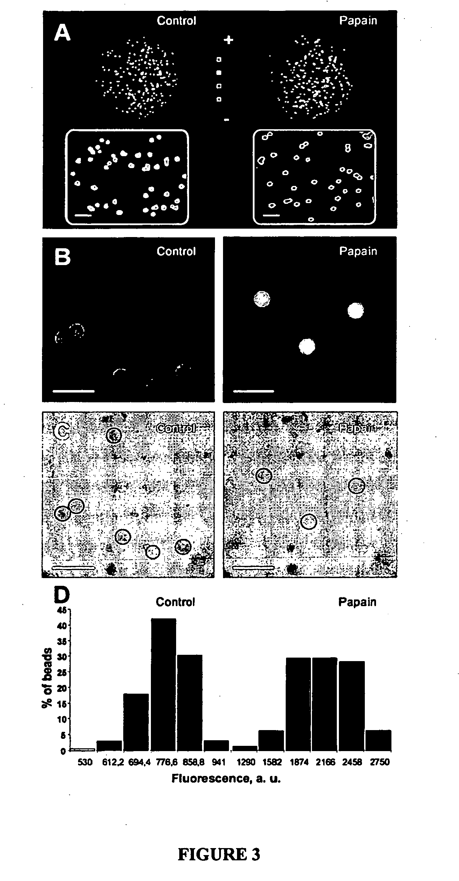

**Figure 3A** illustrates the 5 µm-resolution scanner images of two beads populations : the 20 µm TentaGel beads carrying the fluorogenic papain substrate incubated with 0.5 µM papain (marked "papain") or with NEM-inhibited papain (marked "control").

Automated fluorescence microscopy provides with precise data on brightness variation within the bead population, as illustrated on **Figure 3B** of the fluorescence micrographs of the beads obtained with fluorescein filter set and on **Figure 3C** showing bright field images of the same bead populations as in **Figure 3B****.** These results clearly demonstrate that the beads incubated with papain appeared significantly brighter on scanner image than the control beads.

Individual beads were localized automatically with IMSTAR software and fluorescence intensity was measured. Data were converted into histograms representing a percentage of beads population having the fluorescence in given intensity range. These quantitative data allow detection of small changes in beads brightness, which is impossible by visual examination. These results are illustrated on **Figure 3D** showing histograms of the distribution of the bead brightness in control and in papain-treated bead populations (200 beads in each case).

Thus the analysis of these two bead populations showed that the incubation of the beads with 0.5 µM solution of papain for 2 hours results in ~2.7 fold increase in mean fluorescence with some increase in standard deviation (S.D.) of brightness distribution. This rise in bead brightness was not observed with N-ethylmaleimide-inhibited papain suggesting that it results from the enzymatic cleavage of resin-bound substrate. Therefore, the increase in brightness heterogeneity measured through augmentation of S.D. may be explained by the bead-to-bead variation in substrate cleavage.

## Claims

1. Fluorescent compounds of formula (I): 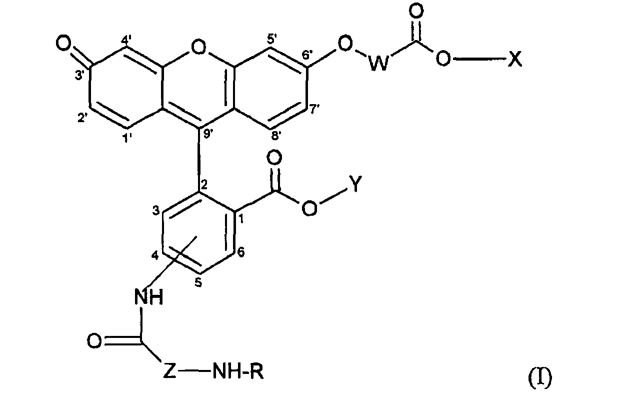 wherein:
W represents a di-radical selected from the group consisting of a linear, branched or cyclic C₁-C₁₂ alkyl, C₂-C₁₂ alkenyl, C₆-C₁₀ aryl, and C₆-C₁₂ alkylaryl or aralkyl,
X is chosen among the group consisting of H, a linear, branched or cyclic C₁-C₁₂ alkyl or C₂-C₁₂ alkenyl, a phenyl, a para-nitro phenyl and a benzyl,
Y represents a radical selected from the group consisting of a linear, branched or cyclic C₁-C₆ alkyl, C₂-C₆ alkenyl, a phenyl, a para-nitro phenyl, a benzyl, C₆-C₁₀ aryl and C₆-C₁₂ alkylaryl or aralkyl optionally containing one or several heteroatoms selected from the group consisting of O, N and S,
Z represents a di-radical selected from the group consisting of C₁-C₁₂ alkyl chains, wherein said chain may be interrupted by one or more of the following functionalities:
an ether bridge -O- , an amino bridge -NH-, an ester function -O-CO-, an amide function -NH-CO-, a sulfur bridge -S-, a urea function -NH-CO-NH-, an oxycarbonyl function -O-CO-O-, C₆-C₁₀ aryl, C₆-C₁₀ heteroaryls optionally containing one or several heteroatoms selected from the group consisting of O, N and S and C₆-C₁₂ alkylaryl or aralkyl,
NH of the phenyl ring is at position 3 to 6 of the ring,
R is an amino protecting group, selected from the group consisting of : Fmoc, t-BuO-CO- (Boc), phenyl, benzyl, acetyl, benzoyl, 4-toluenesulfonyl, 4-methoxy-2,3,6-trimethylbenzenesulfonyl, trityl, 4-methoxytrityl, trifluoroacetyl, benzyloxycarbonyl, allyloxycarbonyl, 2-chlorobenzyloxycarbonyl,
and R, X and Y groups are selected so that R and X can be deprotected independently from each other and without deprotecting Y.

2. The fluorescent compounds of formula (I) according to claim 1, wherein W is a C₁-C₄ alkyl chain di-radical.

3. The fluorescent compounds of formula (I) according to claim 2, wherein W is -CH₂-.

4. The fluorescent compounds of formula (I) according to any one of the preceding claims, wherein X is selected from H, C₁-C₆ alkyl.

5. The fluorescent compounds of formula (I) according to claim 4, wherein X is H.

6. The fluorescent compounds of formula (I) according to claim 4, wherein X is ter-butyl.

7. The fluorescent compounds of formula (I) according to any one of the preceding claims, wherein Y is C₁-C₆ alkyl.

8. The fluorescent compounds of formula (I) according to claim 7, wherein Y is methyl or ethyl.

9. The fluorescent compounds of formula (I) according to claim 8, wherein Z is a C₂-C₈ alkyl chain, optionally comprising one or two functions selected from the group consisting of: an ether bridge, an amide function and an ester function.

10. The fluorescent compounds of formula (I) according to claim 9, wherein Z is selected from the group consisting of -CH₂-CH₂-, -CH₂-CH₂-CH₂-, -CH₂-(CH₂)₂-CH₂-, -CH₂-(CH₂)₃-CH₂-, -CH₂-(CH₂)₄-CH₂-, -CH₂-O-CH₂-CO-NH-CH₂-CH₂-, -CH₂-O-CH₂-CO-NH-(CH₂)₂-CH₂- and -CH₂-O-CH₂-CO-NH-(CH₂)₃-CH₂-.

11. The fluorescent compounds of formula (I) according to any one of the preceding claims, wherein R is a Fmoc group.

12. The fluorescent compounds of formula (I) according to claim 1, selected among the following formulae:

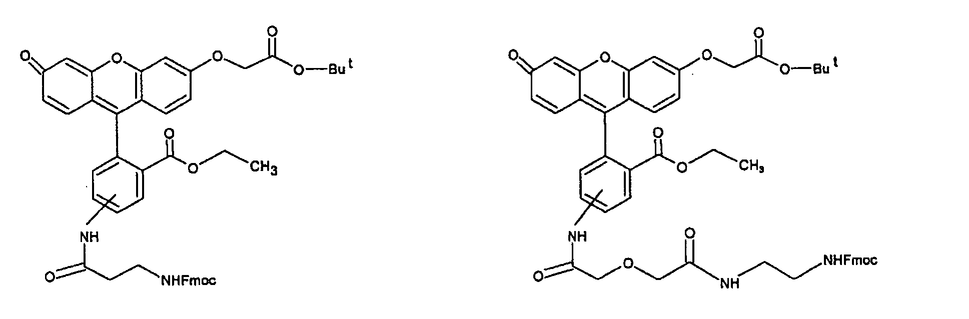

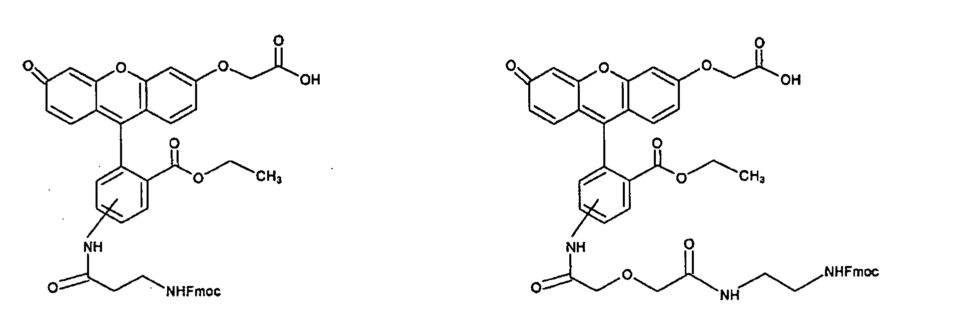

13. A method for producing the fluorescent compounds of formula (I) according to any one of claims 1 to 12, when X is H, comprising at least a step wherein the acidic function grafted on the 6'-hydroxy function of the xanthen ring of compounds of formula (D) is deprotected in order to obtain compounds of formula (E).

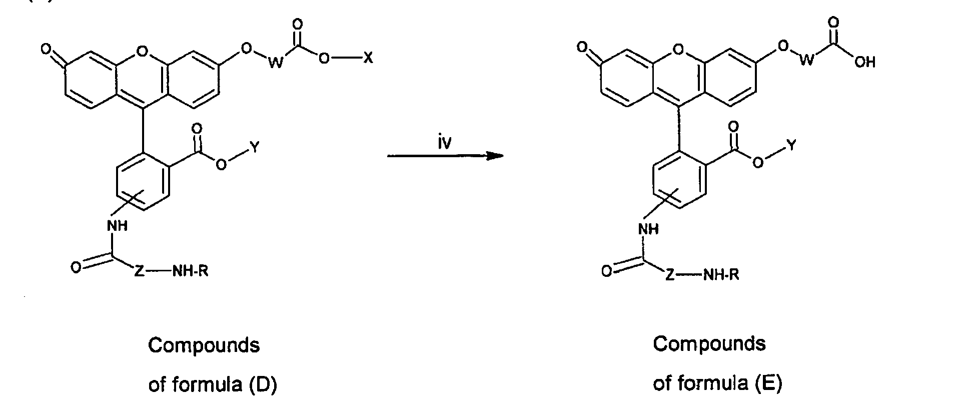

14. The method according to claim 13, wherein compounds of formula (D) are obtained by protecting the compounds of formula (C) on their NH₂ function.

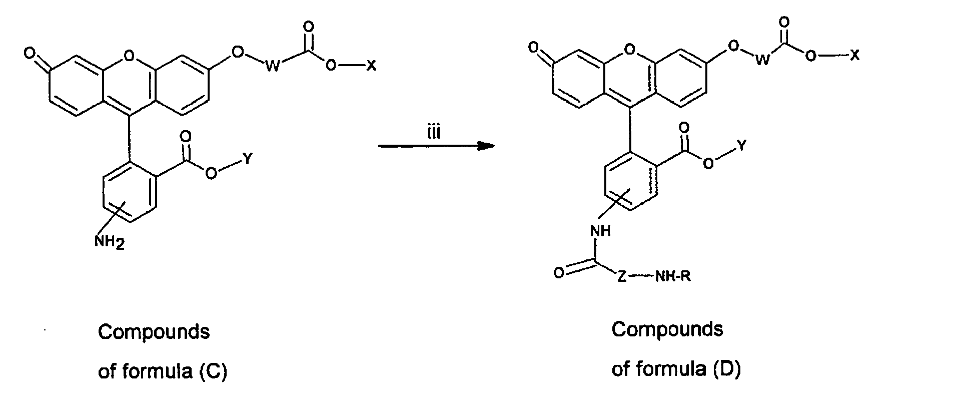

15. The method according to claim 14, wherein Z is an alkyl di-radical, said method comprising a reaction wherein R-NH-Z-COOH is reacted on compound of formula (C) in conditions such that a peptide link is created.

16. The method according to claim 14, wherein Z represents -CH₂-O-CH₂-CO-NH-(CH₂)ₓ-, x being an integer varying from 1 to 6, said method comprising a two steps reaction consisting in reacting the compound of formula C with diglycolic anhydride to obtain a derivative of formula (C) grafted with a -CO-CH₂-O-CH₂-CO-OH group on its amino function, and then in reacting this compound with a R-NH-(CH₂)ₓ-NH₂ molecule in conditions to obtain a coupling between the amino and the carboxylic acid functions.

17. The method according to any one of claim 14 to 16, wherein compounds of formula (C) are obtained by the alkylation of the compounds of formula (B) with an alkyl halogeno-alkylcarboxylate group Hal-W-CO-OX, wherein W and X have the same definition as in formula (I) defined in claim 1, except that X is not H, and Hal represents a halogen atom

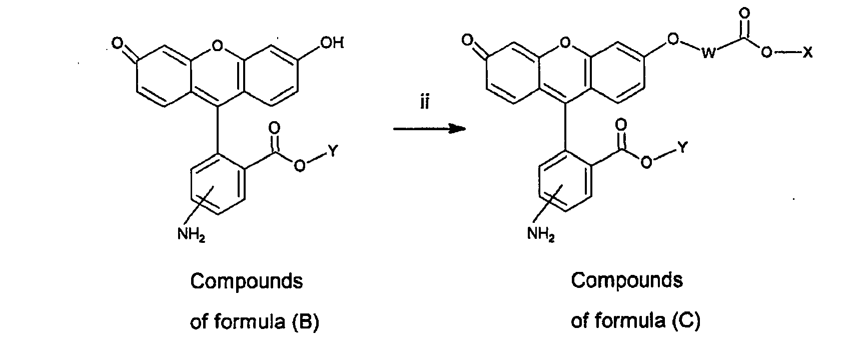

18. The method according to claim 17, wherein Hal is Br.

19. The method according to claim 17 or 18, wherein the alkyl halogeno-alkylcarboxylate group is t-butyl bromoacetate.

20. The method according to any one of claims 17 to 19, wherein the compounds of formula (B) are obtained by esterification of the compounds of formula (A) with alcohol Y-OH, where Y has the same definition as in formula (I) defined in claim 1

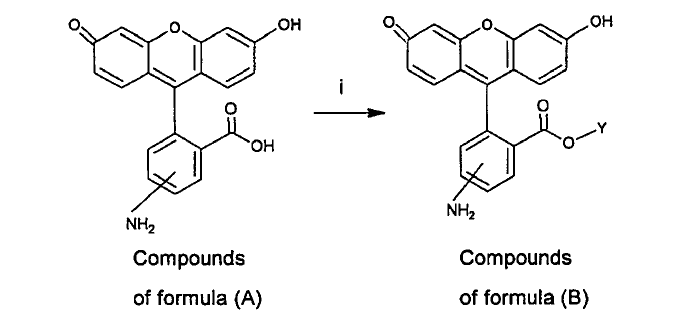

21. Fluorescent compounds of formula (D): 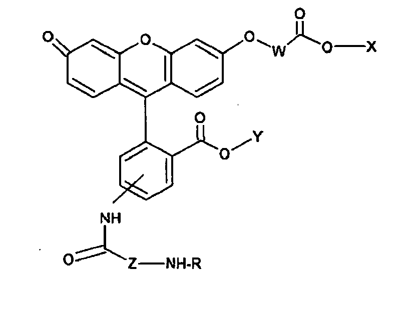 wherein W, X, Y and Z have the same definition as in formula (I) defined in claim 1, except that X is not H.

22. Fluorescent compounds of formula (C): 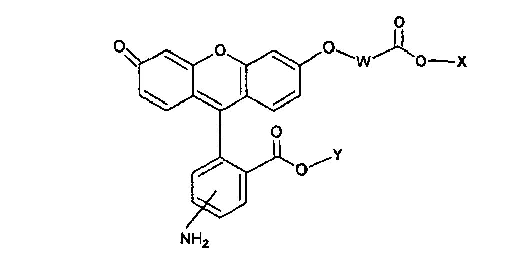 wherein W, X and Y have the same definition as in formula (I) defined in claim 1, except that X is not H.

23. The fluorescent compounds of formula (C) according to claim 22, wherein W is methyl, X is ter-butyl and Y is ethyl.

24. Use of the fluorescent compounds of formula (I) according to anyone of claims 1 to 12 for fluorogenic peptide synthesis.

25. A method for preparing a fluorogenic peptide comprising the steps consisting in:
a) grafting the compound of formula (I) according to anyone of claims 1 to 12 onto a resin with a NH₂ functionality,
b) synthetizing the peptide on the NH₂ group of the compound of formula (I) grafted in step a),
c) grafting a quencher at the NH₂-terminal function of the peptide synthetized in step b).

26. The method according to claim 25, wherein the compound of formula (I) is grafted onto a resin previously coupled to a NH₂-functionalised polar linker.

27. The method according to claim 26, wherein the NH₂-functionalised polar linker is selected in the group consisting in PEG linkers comprising from 2 to 300 PEG units.

28. A compound of formula (II): 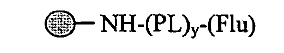 wherein
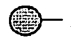 represents a resin,
PL represents a polar linker,
y is an integer selected from 0 and 1,
Flu is a compound of formula (I) according to anyone of claims 1 to 12 grafted by its carboxylic acid function.

29. Kit for peptide synthesis comprising:
- a compound of formula (II) as defined in claim 28, and
- a quencher molecule.

## Patentansprüche

1. Fluoreszierende Verbindungen der Formel (I): 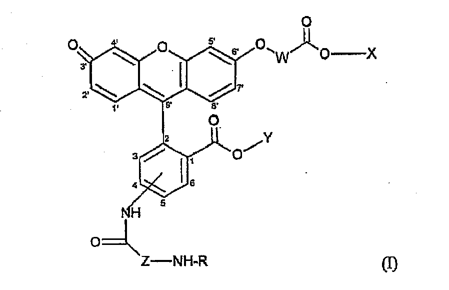 worin:
W ein Diradikal, ausgewählt aus der Gruppe, bestehend aus einem linearen, verzweigten oder cyclischen C₁-C₁₂-Alkyl, C₂-C₁₂-Alkenyl, C₆-C₁₀-Aryl und C₆-C₁₂-Alkylaryl oder -Aralkyl darstellt;
X ausgewählt ist aus der Gruppe, bestehend aus H, einem linearen, verzweigten oder cyclischen C₁-C₁₂-Alkyl oder C₂-C₁₂-Alkenyl, einem Phenyl, einem para-Nitrophenyl und einem Benzyl;
Y ein Radikal darstellt, ausgewählt aus der Gruppe, bestehend aus einem linearen, verzweigten oder cyclischen C₁-C₆-Alkyl, C₂-C₆-Alkenyl, einem Phenyl, einem para-Nitrophenyl, einem Benzyl, C₆-C₁₀-Aryl und C₆-C₁₂-Alkylaryl oder -Aralkyl, das gegebenenfalls ein Heteroatom oder mehrere Heteroatome, ausgewählt aus der Gruppe, bestehend aus O, N und S, enthält;
Z ein Diradikal, ausgewählt aus der Gruppe, bestehend aus C₁-C₁₂-Alkyl-Ketten, wobei die genannte Kette durch eine oder mehrere der folgenden Funktionalitäten unterbrochen sein kann: eine Etherbrücke -O-, eine Aminobrücke -NH-, eine Esterfunktion -O-CO-, eine Amidfunktion -NH-CO-, eine Schwefelbrücke -S-, eine Harnstofffunktion -NH-CO-NH-, eine Oxycarbonylfunktion -O-CO-O-, C₆-C₁₀-Aryl, C₆-C₁₀-Heteroaryle, gegebenenfalls enthaltend ein Heteroatom oder mehrere Heteroatome, ausgewählt aus der Gruppe, bestehend aus O, N und S, und C₆-C₁₂-Alkylaryl oder -Aralkyl, darstellt;
NH des Phenylrings in Position 3 bis 6 des Rings ist;
R eine Aminoschutzgruppe ist, ausgewählt aus der Gruppe, bestehend aus: Fmoc, t-BuO-CO- (Boc), Phenyl, Benzyl, Acetyl, Benzoyl, 4-Toluolsulfonyl, 4-Methoxy-2,3,6-trimethylbenzolsulfonyl, Trityl, 4-Methoxytrityl, Trifluoracetyl, Benzyloxycarbonyl, Allyloxycarbonyl, 2-Chlorbenzyloxycarbonyl; und R-, X- und Y-Gruppen so gewählt sind, dass R und X unabhängig voneinander und ohne Entschützen von Y entschützt werden können.

2. Fluoreszierende Verbindungen der Formel (I) nach Anspruch 1, worin W ein C₁-C₄-Alkylketten-Diradikal ist.

3. Fluoreszierende Verbindungen der Formel (I) nach Anspruch 2, worin W -CH₂- ist.

4. Fluoreszierende Verbindungen der Formel (I) nach einem der vorangehenden Ansprüche, worin X ausgewählt ist aus H, C₁-C₆-Alkyl.

5. Fluoreszierende Verbindungen der Formel (I) nach Anspruch 4, worin X H ist.

6. Fluoreszierende Verbindungen der Formel (I) nach Anspruch 4, worin X tert.-Butyl ist.

7. Fluoreszierende Verbindungen der Formel (I) nach einem der vorangehenden Ansprüche, worin Y C₁-C₆-Alkyl ist.

8. Fluoreszierende Verbindungen der Formel (I) nach Anspruch 7, worin Y Methyl oder Ethyl ist.

9. Fluoreszierende Verbindungen der Formel (I) nach Anspruch 8, worin Z eine C₂-C₈-Alkylkette ist, die gegebenenfalls eine oder zwei Funktion(en), ausgewählt aus der Gruppe, bestehend aus: einer Etherbrücke, einer Amidfunktion und einer Esterfunktion, umfasst.

10. Fluoreszierende Verbindungen der Formel (I) nach Anspruch 9, worin Z ausgewählt ist aus der Gruppe, bestehend aus -CH₂-CH₂-, -CH₂-CH₂-CH₂-, -CH₂-(CH₂)₂-CH₂-, -CH₂-(CH₂)₃-CH₂-, -CH₂-(CH₂)₄-CH₂-, -CH₂-O-CH₂-CO-NH-CH₂-CH₂-, -CH₂-O-CH₂-CO-NH-(CH₂)₂-CH₂- und -CH₂-O-CH₂-CO-NH-(CH₂)₃-CH₂-.

11. Fluoreszierende Verbindungen der Formel (I) nach einem der vorangehenden Ansprüche, worin R eine Fmoc-Gruppe ist.

12. Fluoreszierende Verbindungen der Formel (I) nach Anspruch 1, ausgewählt unter den folgenden Formeln:

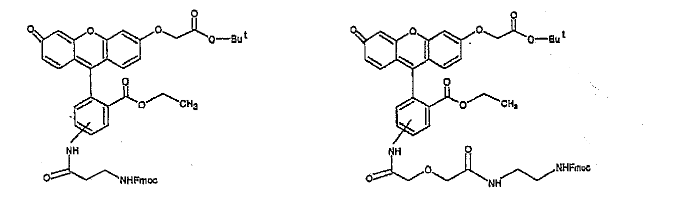

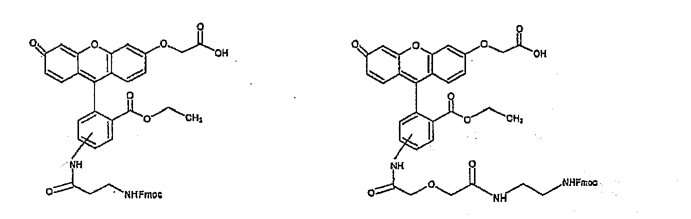

13. Verfahren zur Herstellung der fluoreszierenden Verbindungen der Formel (I) nach einem der Ansprüche 1 bis 12, wenn X H ist, umfassend wenigstens einen Schritt, in dem die saure Funktion, die an die 6'-Hydroxyfunktion des Xanthenrings von Verbindungen der Formel (D) gepfropft ist, entschützt wird, um Verbindungen der Formel (E) zu erhalten.

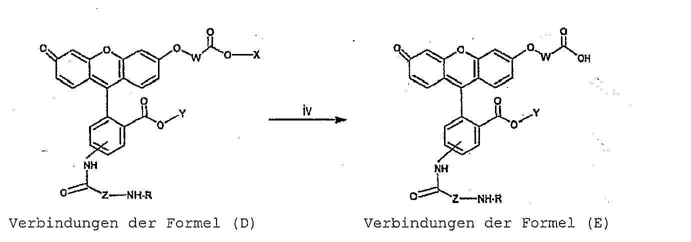

14. Verfahren nach Anspruch 13, wobei Verbindungen der Formel (D) erhalten werden, indem die Verbindungen der Formel (C) an ihrer NH₂-Funktion geschützt werden.

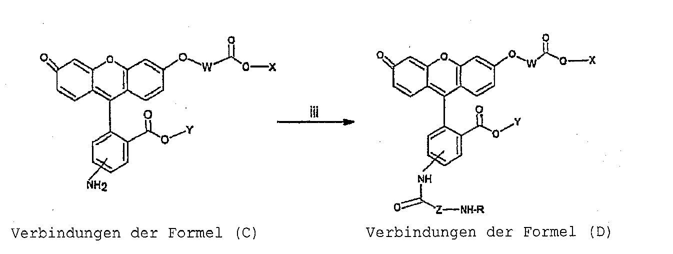

15. Verfahren nach Anspruch 14, wobei Z ein Alkyldiradikal ist, wobei das Verfahren eine Reaktion umfasst, bei der R-NH-Z-COOH mit einer Verbindung der Formel (C) unter solchen Bedingungen umgesetzt wird, dass eine Peptidbindung erzeugt wird.

16. Verfahren nach Anspruch 14, wobei Z -CH₂-O-CH₂-CO-NH-(CH₂)ₓ-, worin x eine ganze Zahl von 1 bis 6 ist, darstellt, wobei das Verfahren eine Zweistufenreaktion umfasst, bestehend aus der Umsetzung der Verbindung der Formel (C) mit Diglycolsäureanhydrid unter Erhalt eines Derivats der Formel (C) das mit einer -CO-CH₂-O-CH₂-CO-OH-Gruppe an seiner Aminfunktion gepfropft ist, und danach einem Umsetzen dieser Verbindung mit einem R-NH-(CH₂)ₓ-NH₂-Molekül unter solchen Bedingungen, dass eine Kopplung zwischen den Amino- und Carbonsäure-Funktionen erhalten wird.

17. Verfahren nach einem der Ansprüche 14 bis 16, wobei Verbindungen der Formel (C) durch die Alkylierung der Verbindungen der Formel (B) mit einer Alkylhalogenalkylcarboxylat-Gruppe Hal-W-CO-OX, worin W und X dieselbe Definition wie in Formel (I), die in Anspruch 1 definiert ist, haben, außer dass X nicht H ist und Hal ein Halogenatom darstellt.

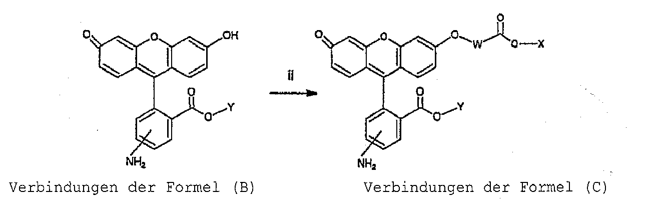

18. Verfahren nach Anspruch 17, wobei Hal Br ist.

19. Verfahren nach Anspruch 17 oder 18, wobei die Alkylhalogenalkylcarboxylat-Gruppe t-Butylbromacetat ist.

20. Verfahren nach einem der Ansprüche 17 bis 19, wobei die Verbindungen der Formel (B) durch Veresterung der Verbindungen der Formel (A) mit Alkohol Y-OH, worin Y dieselbe Definition wie in Formel (I), die in Anspruch 1 definiert ist, hat, erhalten werden.

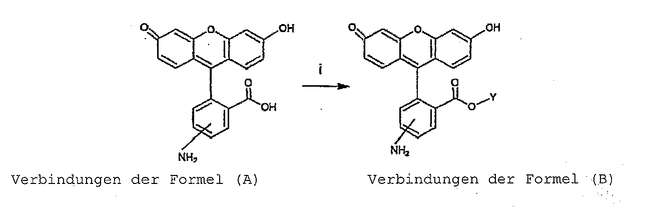

21. Fluoreszierende Verbindungen der Formel (D): 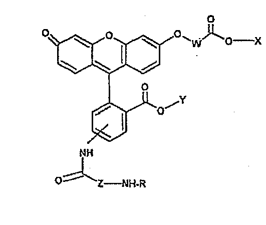 worin W, X, Y und Z dieselbe Definition wie in Formel (I) , die in Anspruch 1 definiert ist, haben, außer dass X nicht H ist.

22. Fluoreszierende Verbindungen der Formel (C): 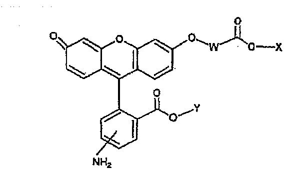 worin W, X und Y dieselbe Definition wie in Formel (I) , die in Anspruch 1 definiert ist, haben, außer dass X nicht H ist.

23. Fluoreszierende Verbindungen der Formel (C) nach Anspruch 22, worin W Methyl ist, X tert.-Butyl ist und Y Ethyl ist.

24. Verwendung der fluoreszierenden Verbindungen der Formel (I) nach einem der Ansprüche 1 bis 12 zur Synthese eines fluorogenen Peptids.

25. Verfahren zur Herstellung eines fluorogenen Peptids, umfassend die Schritte, bestehend aus:
a) Pfropfen der Verbindung der Formel (I) nach einem der Ansprüche 1 bis 12 auf ein Harz mit einer NH₂-Funktionalität,
b) Synthetisieren des Peptids an der NH₂-Gruppe der Verbindung der Formel (I), die in Schritt a) gepropft wurde,
c) Pfropfen eines Quenchers an die NH₂-terminale Funktion des in Schritt b) synthetisierten Peptids.

26. Verfahren nach Anspruch 25, wobei die Verbindung der Formel (I) auf ein Harz gepfropft wird, das vorher an einen NH₂-funktionalisierten polaren Linker gekoppelt worden war.

27. Verfahren nach Anspruch 26, wobei der NH₂-funktionalisierte polare Linker aus der Gruppe ausgewählt wird, die aus PEG-Linkern besteht, die 2 bis 300 PEG-Einheiten umfassen.

28. Verbindung der Formel (II): 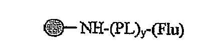 worin
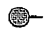 ein Harz darstellt,
PL einen polaren Linker darstellt,
y eine ganze Zahl, ausgewählt aus 0 und 1 ist,
Flu eine Verbindung der Formel (I) nach einem der Ansprüche 1 bis 12 , gepropft durch ihre Carbonsäurefunktion, ist.

29. Kit zur Peptidsynthese, umfassend:
- eine Verbindung der Formel (II), wie sie in Anspruch 28 definiert ist, und
- ein Quencher-Molekül.

## Revendications

1. Composés fluorescents de formule (I) : 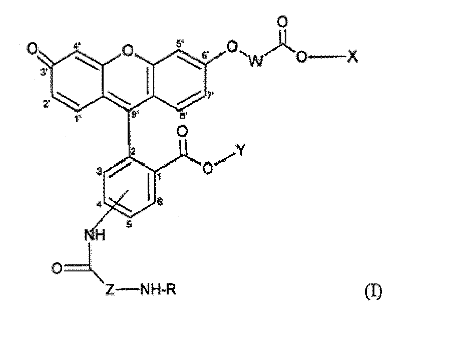 dans laquelle :
W représente un di-radical choisi dans le groupe constitué de : un groupe alkyle en C₁ à C₁₂, alcényle en C₂ à C₁₂, aryle en C₆ à C₁₀ et alkylaryle ou aralkyle en C₆ à C₁₂ linéaire ou ramifié ou cyclique,
X est choisi dans le groupe constitué de : H, un groupe alkyle en C₁ à C₁₂ ou alcényle en C₂ à C₁₂ linéaire, ramifié ou cyclique, un groupe phényle, paranitrophényle et benzyle,
Y représente un radical choisi dans le groupe constitué de : un groupe alkyle en C₁ à C₆, alcényle en C₂ à C₆ linéaire, ramifié ou cyclique, un groupe phényle, paranitrophényle, benzyle, aryle en C₆ à C₁₀ et alkylaryle ou aralkyle en C₆ à C₁₂ contenant éventuellement un ou plusieurs hétéro-atomes choisis dans le groupe constitué de : O, N et S,
Z représente un di-radical choisi dans le groupe constitué de : des chaînes alkyle en C₁ à C₁₂, où lesdites chaînes peuvent être interrompues par une ou plusieurs des fonctionnalités suivantes : un pont éther -O-, un pont amino -NH-, une fonction ester -O-CO-, une fonction amide -NH-CO-, un pont soufre -S-, une fonction urée -NH-CO-NH-, une fonction oxycarbonyle -0-CO-O-, un groupe aryle en C₆ à C₁₀, des groupes hétéroaryle en C₆ à C₁₀ contenant éventuellement un ou plusieurs hétéro-atomes choisis dans le groupe constitué de : O, N et S et un groupe alkylaryle ou aralkyle en C₆ à C₁₂,
NH du cycle phényle est en position 3 à 6 du cycle,
R est un groupe amino protecteur choisi dans le groupe constitué de : Fmoc, t-BuO-CO- (Boc), phényle, benzyle, acétyle, benzoyle, 4-toluène-sulfonyle, 4-méthoxy-2,3,6-triméthylbenzènesulfonyle, trityle, 4-méthoxytrityle, trifluoroacétyle, benzyloxycarbonyle, allyloxycarbonyle, 2-chlorobenzyloxycarbonyle,
et les groupes R, X et Y sont choisis de telle sorte que R et X peuvent être déprotégés indépendamment les uns des autres et sans déprotéger Y.

2. Composés fluorescents de formule (I) selon la revendication 1, dans lesquels W est un di-radical à chaîne alkyle en C₁ à C₄.

3. Composés fluorescents de formule (I) selon la revendication 2, dans lesquels W est -CH₂-.

4. Composés fluorescents de formule (I) selon l'une quelconque des revendications précédentes, dans lesquels X est choisi parmi H, un groupe alkyle en C₁ à C₆.

5. Composés fluorescents de formule (I) selon la revendication 4, dans lesquels X est H.

6. Composés fluorescents de formule (I) selon la revendication 4, dans lesquels X est un groupe tert-butyle.

7. Composés fluorescents de formule (I) selon l'une quelconque des revendications précédentes, dans lesquels Y est un groupe alkyle en C₁ à C₆.

8. Composés fluorescents de formule (I) selon la revendication 7, dans lesquels Y est un groupe méthyle ou éthyle.

9. Composés fluorescents de formule (I) selon la revendication 8, dans lesquels Z est une chaîne alkyle en C₂ à C₈ comprenant éventuellement une ou deux fonctions choisies dans le groupe constitué de : un pont éther, une fonction amide et une fonction ester.

10. Composés fluorescents de formule (I) selon la revendication 9, dans lesquels Z est choisi dans le groupe constitué de : -CH₂-CH₂-, -CH₂-CH₂-CH₂-, -CH₂-(CH₂)₂-CH₂-, -CH₂-(CH₂)₃-CH₂-, -CH₂-(CH₂)₄-CH₂-, -CH₂-O-CH₂-CO-NH-CH₂-CH₂-, -CH₂-O-CH₂-CO-NH-(CH₂)₂-CH₂- et -CH₂-O-CH₂-CO-NH-(CH₂)₃-CH₂-.

11. Composés fluorescents de formule (I) selon l'une quelconque des revendications précédentes, dans lesquels R est un groupe Fmoc.

12. Composés fluorescents de formule (I) selon la revendication 1, choisis parmi les formules suivantes :

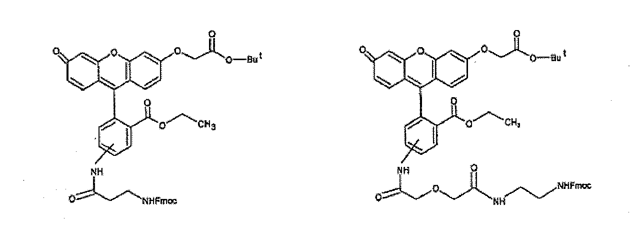

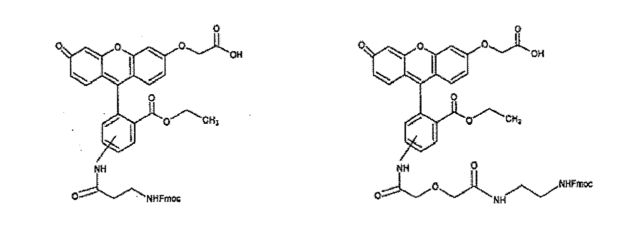

13. Procédé de production des composés fluorescents de formule (I) selon l'une quelconque des revendications 1 à 12, dans lesquels X est H, comprenant au moins une étape dans laquelle la fonction acide greffée sur la fonction 6'-hydroxy du cycle xanthène des composés de formule (D) est déprotégée afin d'obtenir des composés de formule (E).

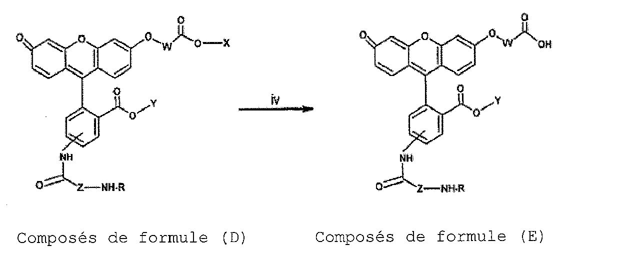

14. Procédé selon la revendication 13, dans lequel les composés de formule (D) sont obtenus en protégeant les composés de formule (C) sur leur fonction NH₂.

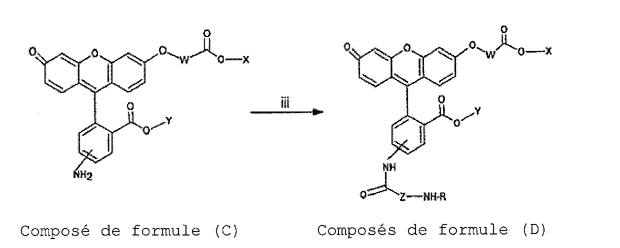

15. Procédé selon la revendication 14, dans lequel Z est un di-radical alkyle, ledit procédé comprenant une réaction dans laquelle on fait réagir R-NH-Z-COOH sur un composé de formule (C) dans des conditions telles qu'un lien peptide soit créé.

16. Procédé selon la revendication 14, dans lequel Z représente -CH₂-O-CH₂-CO-NH-(CH₂)ₓ-, x étant un nombre entier de 1 à 6, ledit procédé comprenant une réaction en deux étapes consistant en la réaction du composé de formule C avec un anhydride diglycolique pour obtenir un dérivé de formule (C) greffé avec un groupe -CO-CH₂-O-CH₂-CO-OH sur sa fonction amino, puis en faisant réagir ce composé avec une molécule R-NH-(CH₂)ₓ-NH₂ dans des conditions permettant d'obtenir un couplage entre les fonctions amino et acide carboxylique.

17. Procédé selon l'une quelconque des revendications 14 à 16, dans lequel les composés de formule (C) sont obtenus par l'alkylation des composés de formule (B) avec un groupe Hal-W-CO-OX alkyl-halogéno-alkylcarboxylate, dans lequel W et X ont la même définition que dans la formule (I) définie dans la revendication 1, excepté le fait que X n'est pas H et Hal représente un atome d'halogène.

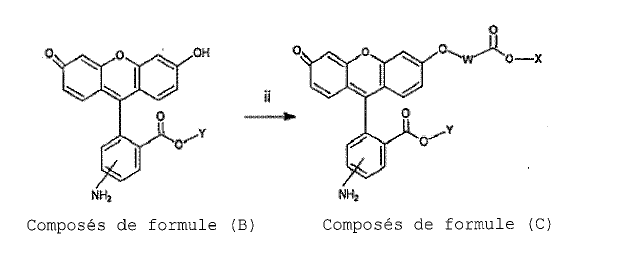

18. Procédé selon la revendication 17, dans lequel Hal est Br.

19. Procédé selon la revendication 17 ou 18, dans lequel le groupe alkyl-halogéno-alkylcarboxylate est le t-butyl-bromo-acétate.

20. Procédé selon l'une quelconque des revendications 17 à 19, dans lequel les composés de formule (B) sont obtenus par estérification des composés de formule (A) avec un alcool Y-OH, où Y a la même définition que dans la formule (I) définie dans la revendication 1

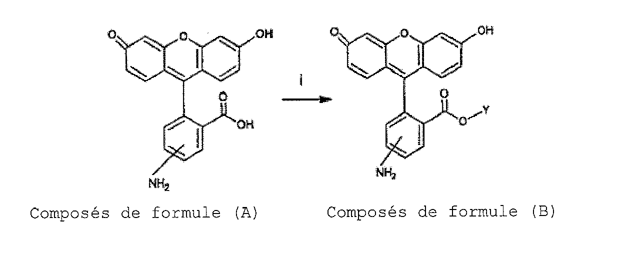

21. Composés fluorescents de formule (D) : 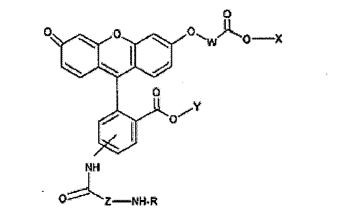 dans laquelle W, X, Y et Z ont la même définition que dans la formule (I) définie dans la revendication 1, excepté le fait que X n'est pas H.

22. Composés fluorescents de formule (C) : 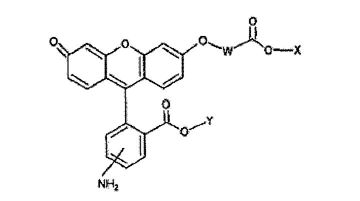 dans laquelle W, X et Y ont la même définition que dans la formule (I) définie dans la revendication 1, excepté le fait que X n'est pas H.

23. Composés fluorescents de formule (C) selon la revendication 22, dans lesquels W est un groupe méthyle, X est un groupe tert-butyle et Y est un groupe éthyle.

24. Utilisation des composés fluorescents de formule (I) selon l'une quelconque des revendications 1 à 12 pour la synthèse de peptides fluorogènes.

25. Procédé de préparation d'un peptide fluorogène comprenant les étapes consistant à :
a) greffer le composé de formule (I) selon l'une quelconque des revendications 1 à 12 sur une résine comportant une fonctionnalité NH₂,
b) synthétiser le peptide sur le groupe NH₂ du composé de formule (I) greffé à l'étape a),
c) greffer un désactiveur au niveau de la fonction NH₂-terminale du peptide synthétisé à l'étape b).

26. Procédé selon la revendication 25, dans lequel le composé de formule (I) est greffé sur une résine couplée précédemment à un segment de liaison polaire fonctionnalisé par NH₂.

27. Procédé selon la revendication 26, dans lequel le segment de liaison polaire fonctionnalisé par NH₂ est choisi dans le groupe comprenant des segments de liaison PEG comprenant de 2 à 300 unités PEG.

28. Composé de formule (II) : 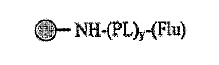 dans laquelle
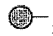 représente une résine,
PL représente un segment de liaison polaire,
y est un nombre entier de 0 à 1,
Flu est un composé de formule (I) selon l'une quelconque des revendications 1 à 12 greffé par sa fonction acide carboxylique.

29. Kit de synthèse peptidique comprenant :
- un composé de formule (II) tel que défini dans la revendication 28, et
- une molécule de désactivation.
